(19) Europäisches Patentamt
European Patent Office
Office européen des brevets

(11) Veröffentlichungsnummer: **0 105 030 B1**

(12) **EUROPÄISCHE PATENTSCHRIFT**

(45) Veröffentlichungstag der Patentschrift: **17.12.86**

(21) Anmeldenummer: **83810376.0**

(22) Anmeldetag: **22.08.83**

(51) Int. Cl.⁴: **A 01 N 43/54**, A 01 N 47/02, A 01 N 53/00, D 06 M 13/40 // (A01N53/00, 47:02, 43:54)

(54) **Motten- und Käferschutzmittel.**

(30) Priorität: **26.08.82 CH 5081/82**

(43) Veröffentlichungstag der Anmeldung: **04.04.84 Patentblatt 84/14**

(45) Bekanntmachung des Hinweises auf die Patenterteilung: **17.12.86 Patentblatt 86/51**

(84) Benannte Vertragsstaaten: **BE CH DE FR GB IT LI**

(56) Entgegenhaltungen:
**EP-A-0 007 541**
**DE-A-2 936 457**

(73) Patentinhaber: **CIBA- GEIGY AG, Klybeckstrasse 141, CH- 4002 Basel (CH)**

(72) Erfinder: **de Sousa, Bernardo, Dr., Paradiesstrasse 15, CH- 4125 Riehen (CH)**
Erfinder: **Schmid, Werner, Keltenweg 40, CH- 4125 Riehen (CH)**
Erfinder: **Artz, Klaus, Dr., Ahornstrasse 3, CH- 4132 Muttenz (CH)**

EP 0 105 030 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein Mittel zum Schützen von Keratinmaterial gegen den Befall durch keratinfressende Schädlinge, insbesondere ein Mittel zum Schützen von Wolle, Pelzen und Federn gegen den Befall und Fraßschäden durch Motten- und Käferlarven sowie ein Verfahren zum Schützen der genannten Materialien vor dem Befall durch keratinfressende Schädlinge.

Es ist bekannt, dass gewisse synthetische Pyrethroide zur Bekämpfung von keratinfressenden Schädlingen verwendet werden können. Siehe z.B. J. Text. Inst. 1976, No. 3, Vol. 67, 77; . DE-A 29 23 217; US-A 4,219,593; EP-B 11 789. Diese Pyrethroide wirken sehr gut gegen Mottenlarven, während die Wirkung gegen Larven von Pelz- und Teppichkäfern weniger ausgeprägt ist. Aus der US-A 4,283,444 ist ausserdem bekannt, dass gewisse 5-Phenylcarbamoylbarbitursäuren ebenfalls zur Bekämpfung von Keratinschädlingen eingesetzt werden können. Diese zeigen eine besonders gute Wirkung gegenüber Pelz- und Teppichkäferlarven. In der US-A 4,283,444 sind auch Mittel beschrieben, die eine Kombination aus den genannten synthetischen Pyrethroiden und 5-Phenylcarbamoylbarbitursäuren enthalten mit deren Hilfe man Keratinmaterialien (z.B. Wolle) ausgezeichnet gegen den Befall durch Keratinschädlinge schützen kann.

Es wurde nun überraschenderweise gefunden, dass mit Hilfe eines Mittels (Kombinationspräparates), das ein synthetisches Pyrethroid und ein 5-Phenylcarbamoylbarbiturat, das im Phenylring spezifische Substituenten aufweist, enthält, auf Keratinmaterialien, insbesondere Wolle, ganz besonders hohe Schutzwirkungen gegen die genannten Schädlinge erzielt bzw. für gleiche Schutzwirkung geringere Mengen der Wirkstoffkombination benötigt werden als bei Verwendung der oben erwähnten bekannten Mittel.

Das erfindungsgemässe Mittel ist dadurch gekennzeichnet, dass es (A) ein oder mehrere 5-Phenylcarbamoylbarbiturat(e) der Formel

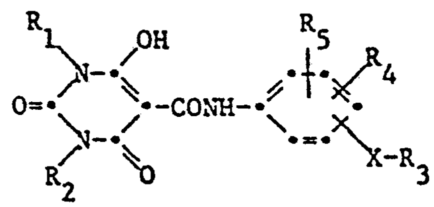

(1)

sowie deren tautomere Formen und Salze, worin $R_1$ und $R_2$ unabhängig voneinander $C_1$-$C_4$-Alkyl, $C_3$-$C_6$-Cycloalkyl, $C_2$-$C_4$-Alkenyl, Benzyl, Phenyl oder mit 1 bis 3 Substituenten aus der Gruppe Halogen, $C_1$-$C_4$-Alkyl, $C_1$-$C_4$-Alkoxy und Nitro substituiertes Phenyl,

$R_3$ $C_1$-$C_4$-Halogenalkyl, Phenyl oder durch 1-3 Substituenten aus der Gruppe $C_1$-$C_4$-Halogenalkyl, Nitro, Halogen und $C_1$-$C_4$-Alkyl substituiertes Phenyl,

$R_4$ und $R_5$ unabhängig voneinander Wasserstoff, Halogen, $C_1$-$C_4$-Halogen-alkyl, $C_1$-$C_4$-Alkyl, $C_2$-$C_4$-Alkenyl, $C_3$-$C_6$-Cycloalkyl, Methoxy oder Nitro und

X Sauerstoff oder Schwefel bedeuten, wobei X-$R_3$ gemeinsam mit $R_4$ oder $R_5$ in ortho-Stellung auch für die Gruppe $-O-CF_2-O-CF_2-$stehen kann,

und

(B) ein oder mehrere synthetische(s) Pyrethroid(e) der allgemeinen Formel

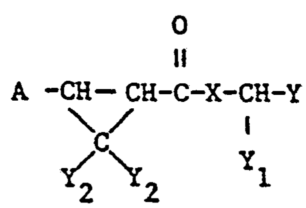

(2),

worin

A 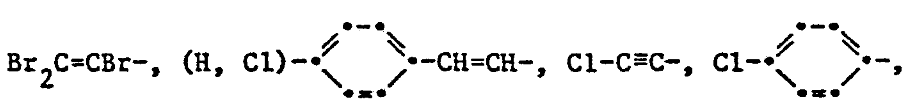

$(CH_3)_3C-O-$,

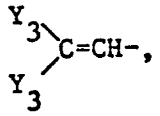

worin $Y_3$ für Cl, Br, $CF_3$, F oder $C_1$-$C_4$-Alkyl steht,
$CH_2=CH-CH_2-O-$ oder

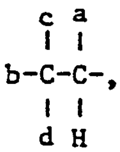

worin a, b, c und d unabhängig voneinander für Cl, Br oder F stehen, wobei c und d auch für Methyl stehen können,
X Sauerstoff oder Schwefel, $Y_1$ Wasserstoff, CN, $CH_3$, $C_2H_5$, i-$C_3H_7$,

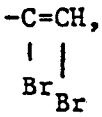, $-C\equiv CH$, $-C\equiv C-CH_3$, $-C\equiv C-C_6H_5$, $-CH=CH-CH_3$, $-CH_2-CH=CH_2$,

$-CH=CH_2$ oder $-CH_2-CH=CHCl$,

$Y_2$ Methyl oder beide $Y_2$ zusammen die Ergänzung zu einem Cyclopropan-, Cyclobutan- oder Cyclopentanring und

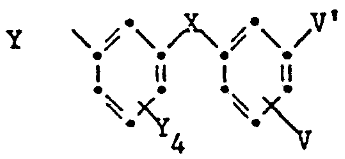,

worin $Y_4$ für Wasserstoff oder Fluor und V für Wasserstoff, Cl, Br, F, $CH_3$ oder $NO_2$ und V' für Wasserstoff stehen, wobei V' für den Fall dass V Wasserstoff bedeutet, auch für $CF_3$ stehen kann, und X wie oben definiert ist; ferner Y

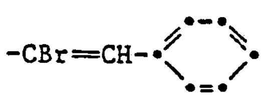, 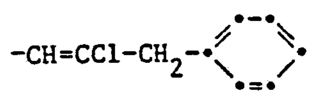,

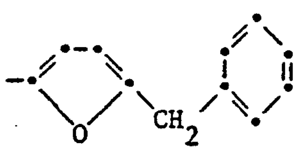, 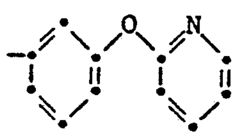 oder 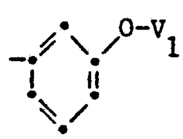,

bedeuten,
worin $V_1$ für $-CH_2-CH=CH_2$, $-CH_2-C\equiv CH$, $-CH_2-CH=CH-CH_3$,

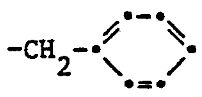 , 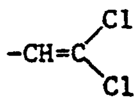 ,

$-CF=CFCl$ oder $-CF=CF_2$ steht, oder der allgemeinen Formel

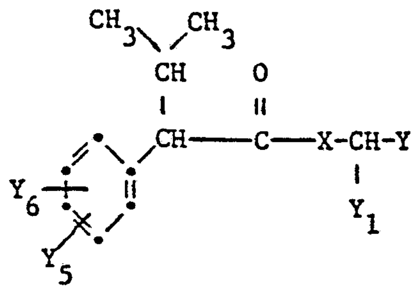 (2a),

worin X, Y und $Y_1$ wie in Formel (2) definiert sind, $Y_5$ Wasserstoff, $CH_3$, Cl, $NO_2$, $OCH_3$, $OCH(CH_3)_2$, $-OCH_2C{\equiv}CH_2$ oder $-OCH_2CH=CH_2$ und $Y_6$ Wasserstoff, $CH_3$, Cl, Br oder F oder $Y_5$ und $Y_6$ in ortho-Stellung gemeinsam die Ergänzung zu einem ankondensierten Benzolring bedeuten, enthält.

Beispielsweise enthalten erfindungsgemässe Mittel solche Verbindungen der Formel (1) worin $R_1$ und $R_2$ unabhängig voneinander $C_1$-$C_4$-Alkyl oder $C_2$-$C_4$-Alkenyl,

$R_3$ $C_1$-$C_4$-Halogenalkyl oder durch $C_1$-$C_4$-Halogenalkyl substituiertes Phenyl,

$R_4$ und $R_5$ unabhängig voneinander Wasserstoff, Halogen, $C_1$-$C_4$-Halogen-alkyl, Methoxy oder Nitro bedeuten und für den Fall, dass $X$-$R_3$ für 4-Trifluor-methylphenoxy steht, zusätzlich in 2- und/oder 6-Stellung $C_1$-$C_4$-Alkyl $C_2$-$C_4$-Alkenyl oder Cyclopropyl stehen können, und X Sauerstoff oder Schwefel bedeuten, wobei $X$-$R_3$ gemeinsam mit $R_4$ oder $R_5$ in ortho-Stellung auch für die Gruppe $-O-CF_2-O-CF_2-$ stehen kann.

Die 5-Phenylcarbamoylbarbiturate der Formel (1) (Komponente (A)) liegen in verschiedenen tautomeren Formen (Keto/Enol-Tautomerie) etwa nach dem folgenden Schema vor:

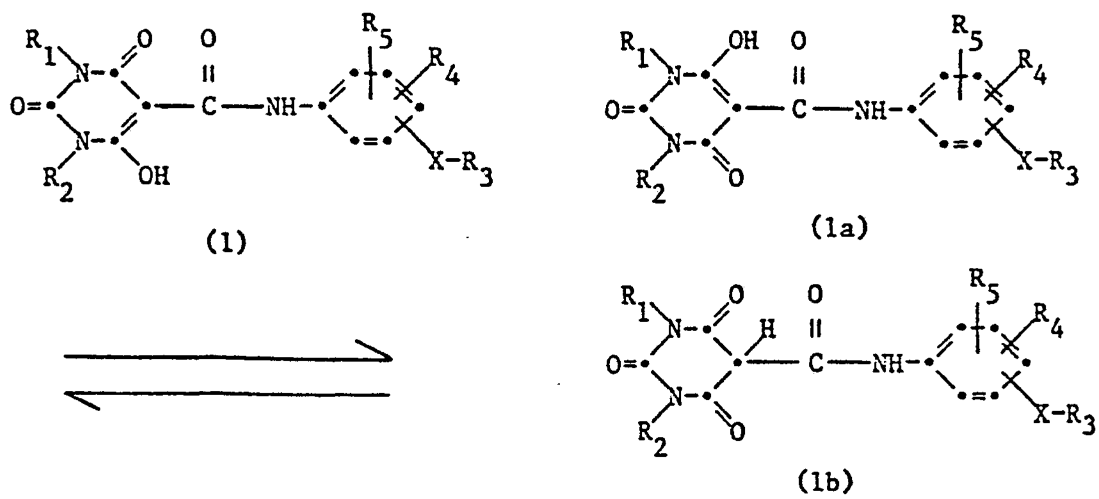

(1) (1a) (1b)

Alle tautomeren Formen bzw. deren Mischungen können im erfindungsgemässen Mittel eingesetzt werden. Die einzelnen Formeln umfassen daher auch die jeweils möglichen tautomeren Formen.

Die Wirkstoffe der Formel (1) können im erfindungsgemässen Mittel auch in Form ihrer Salze eingesetzt werden. Unter den Salzen sind die Alkalimetall-, Ammonium- oder Aminsalze besonders zu erwähnen, wobei Natrium-, Kalium-, Ammonium- oder Alkylamin-, insbesondere Triäthylaminsalze bevorzugt sind.

Von den 5-Phenylcarbamoylbarbitursäureverbindungen der Formel (1) werden bevorzugt solche eingesetzt, in denen X Sauerstoff bedeutet.

In bevorzugten Verbindungen der Formel (1) steht der Substituent $-X-R_3$ in ortho- oder para-Stellung. $R_1$ und $R_2$ bedeuten in Formel (1) vorzugsweise $C_1$-$C_4$-Alkyl, insbesondere Methyl oder Aethyl.

Die Substituenten $R_4$ und/oder $R_5$ bedeuten vorzugsweise Wasserstoff, $C_1$-$C_4$-Alkyl oder Halogen, insbesondere Chlor.

Unter "Halogen" ist insbesondere Fluor, Chlor oder Brom zu verstehen, wobei Chlor und Fluor bevorzugt sind.

Unter den $C_1$-$C_4$-Halogenalkylresten (Substituenten $R_3$, $R_4$, $R_5$ in Formel (1)) sind jene bevorzugt, die 1 oder 2 C-Atome und 1 bis 5 gleiche oder verschiedene Halogenatome, insbesondere Fluor- oder Chloratome enthalten. Beispiele hierfür sind: 1,1,2-Trifluor-2-chloräthyl, 1,1,2,2-Tetrafluoräthyl 1,1,2,2-Tetrafluor-2-chloräthyl, Chlordifluormethyl, Dichlorfluormethyl, Dichlormethyl, Difluormethyl, Trichlormethyl und insbesondere Trifluormethyl.

In weiteren bevorzugten Verbindungen der Formel (1) steht $R_3$ für einen $C_1$-$C_4$-Halogenalkylrest, besonders für einen der vorstehend beispielhaft aufgezählten, und X steht für Sauerstoff.

Besonders gute Wirkung zeigen auch jene erfindungsgemässen mittel, die als Komponente A eine Verbindung der Formel (1) enthalten, worin X Sauerstoff und $R_3$ einen mit 1 bis 3, vorzugsweise 1 bis 2, Substituenten aus der Gruppe $C_1$-$C_4$-Halogenalkyl, Halogen oder $C_1$-$C_4$-Alkyl substituierten Phenylrest bedeuten, wobei der Rest $-X-R_3$ vorzugsweise in ortho- oder para-Stellung steht. Hervorzuheben sind dabei noch jene Mittel, in deren Komponente (A) der Formel (1) $R_4$ und $R_5$ in 2- und 6-Stellung stehen und Wasserstoff oder $C_1$-$C_4$-Alkyl bedeuten und $-X-R_3$ für 4-Trifluormethylphenoxy steht.

Als bevorzugte Komponenten (B) können solche der oben definierten Formel (2) eingesetzt werden, worin A einen Rest der Formel

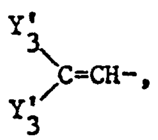

worin $Y'_3$ für Br, Cl oder $CH_3$ steht, X Sauerstoff und Y

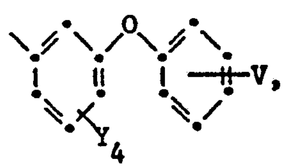

bedeuten. $Y_2$ bedeutet in Formel (2) vorzugsweise $CH_3$ und $Y_1$ Wasserstoff, CN, $CH_3$, $-CH=CH_2$, $-C\equiv CH$ oder $-C\equiv C-CH_3$, insbesondere Wasser-Stoff oder CN.

In praktisch wichtigen erfindungsgemässen Mitteln ist die Komponente (B) eine Verbindung aus der Klasse der 3-(2',2'-Dihalogenvinyl)-2,2-dimethylcyclopropan-carbonsäure-3''-phenoxybenzylester, insbesondere eine solche der Formel

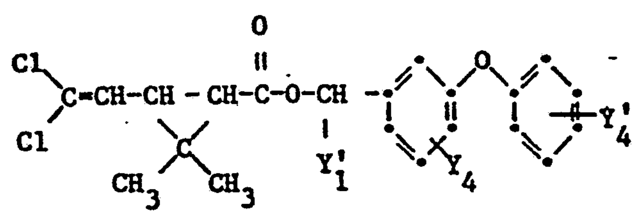 (3),

worin Y' Wasserstoff, Cyano, $-CH=CH_2$ oder $-C\equiv C-CH_3$ und $Y_4$ und $Y'_4$ unabhängig voneinander Wasserstoff oder Fluor bedeuten. Der Trivialname der Verbindung der Formel (3) mit $Y'_1=Y_4=Y'_4=$ Wasserstoff ist Permethrin, jener mit $Y'_1=CN$ und $Y_4=Y'_4=$ Wasserstoff ist Cypermethrin.

Die erfindungsgemässen Mittel können ausschliesslich aus den Komponenten (A) und (B) bestehen oder sie

können zusätzlich noch übliche Träger bzw. Formulierungsmittel wie Lösungsmi-ttel, Wasser, Säuren, Basen, Tenside, Netz-, DisPergier- und/oder Emulgietmittel enthalten.

Als Lösungsmittel kommen beispielsweise organische Lösungsmittel wie z.B. aliphatische und alicyclische Alkohole, Ketone, Kohlenwasserstoffe wie Benzol, Xylole, Toluol, Benzine, ferner chlorierte und fluorierte Kohlenwasserstoffe, insbesondere Propylenglykol, Methoxyäthenol, Aethoxyäthenol oder Dimethylformamid in Betracht, gegebenenfalls in Mischung mit Wasser.

Als weitere Formulierungsmittel kommen etwa die üblichen, als Netz-, Dispergier- und/oder Emulgiermittel verwendeten oberflächenaktiven Substanzen in Betracht, beispielsweise solche, die weiter unten als Zusätze für Applikationsbäder im erfindungsgemässen Verfahren angeführt sind.

Besonders vorteilhaft enthalten die erfindungsgemässen Mittel Formulierungsbestandteile, die zu einer lagerstabilen, direkt verwendbaren Formulierung führen. Derartige Formulierungsbestandteile sind in der EP-A 7C 335 beschrieben (siehe dort Komponenten C bis G). Die vorliegende Erfindung betrifft daher auch Mittel, die die Komponenten (A) und (B) sowi-e die in der genannten EP-A beschriebenen Komponenten C bis G enthalten. Diese dort beschriebenen Komponenten und ihre bevorzugten Vertreter sowie ihre relativen Mengenverhältnisse werden zum Bestandteil der vorliegenden Beschreibung erklärt.

Die Pyrethroide der Formel (2) (Komponente (B)) sind bekannt. Siehe dazu z.B. die Literaturangaben auf Seite 1. Die 5-Phenylcarbamoyl-barbiturate der Formel (1) sind teilweise aus der EP-A 7541 bekannt bzw. sie können nach den dort angegebenen Verfahren erhalten werden. Die Herstellung einiger dort nicht genannten Verbindungen der Formel (1) wird in den Herstellungsvorschriften des nachfolgenden Beispielteiles beschrieben.

Das Mischungsverhältnis der heiden Wirkstoffkomponenten (A) und (B) in den erfindungsgemässen Mitteln (Wirkstoffkombinationen) kann in weiten Grenzen schwanken; es kann beispielsweise zwischen 1:4 und C:l, vorzugsweise zwischen 1:2 und 2:1 liegen. In besonders bevorzugten erfindungsgemässen Mitteln beträgt dieses Verhältnis etwa 1:1.

Die erfindungsgemässen Mittel können zum Schützen von Keratinmaterial gegen keratinfressende Insekten eingesetzt werden, wie z.B. gegen keratinfressende Larven von Lepidoptera, z.B. Tineola spec. und Tinea spec. sowie gegen keratinfressende Larven von Coleoptera, z.B. Anthrenus spec. und Attagenus spec. Die Mittel eignen sich vorzüglich zum Schützen von keratinischen bzw. keratinhaltigem Material gegen Insektenfrass, insbesondere zur wasch- und lichtechten Ausrüstung gegen Insekten, insbesondere zur Motten- und Käferechtausrüstung von derartigen Materialien. Es kann keratinisches bzw. keratinhaltiges Material sowohl in rohem als auch in verarbeitetem Zustand ausgerüstet werden, z.B. rohe oder verarbeitete Schafwolle, Produkte aus anderen Tierhaaren, Felle, Pelze und Federn.

Praktisch besonders wichtig ist die Wirksamkeit der erfindungsgemässen Mittel gegen die Larven der Kleidetmotte (Tineola bisselliella), der Pelzmotte (Tinea pellionella) und der Samenmotte (Hofmannophila pseudopretella) sowie gegen die Larven der Pelz- und Teppichkäfer (Attagenus spec. bzw. Anthrenus spec.), z.B. des Wollkraut-Blütenkäfers (Anthrenus verbasci), des Bibernell-Blütenkäfers (Anthrenus pimpinellae), des Gemeinen Teppichkäfers (Anthrenus scrophilariae), des Bebänderten Teppichkäfers (Anthrenus fasciatus), des Gefleckten Pelzkäfers (Attagenus Pellio) und vor allem des Dunklen Pelzkäfers (Attagenus piceus) und des Teppichkäfers (Anthrenus vorax).

Bevorzugt werden die erfindungsgemässen Mittel einerseits zum Schützen von Textilien aus Wolle, z.B. von Wolldecken, Wollteppichen, Wollwäsche, Wollkleidern und Wirkwaren bzw. wollhaltigen Textilien, wie Mischgeweben, deren eine Komponente Wolle ist, z.B. Mischgeweben aus Wolle und anderen Naturfasern, vorzugsweise Baumwolle, oder aus Wolle und Kunstfasern, andererseits auch zum Schützen von Pelzen und Fellen vor dem Befall durch die erwähnten Schädlinge eingesetzt.

Die vorliegende Erfindung betrifft auch ein Verfahren zum Schützen von Keratinmaterial, insbesondere von Wolltextilien, vor dem Befall durch Keratinschädlinge, z.B. Motten- und Käferlarven, das dadurch gekennzeichnet ist, dass man das zu schützende Material mit einer Kombination aus einem oder mehreren 5-Phenylcarbamoylbarbiturat(en) der Formel (1) und einem oder mehreren synthetischen Pyrethroid(en), vorzugsweise solchen der Formeln (2) und (2a), behandelt. Zu diesem Zweck wird in der Regel diese Wirkstoffkombination, die als erfindungsgemässes Mittel gegebenenfalls noch übliche Träger bzw. Formulierungshilfsmittel enthalten kann, in eine Applikationsflotte eingebracht, der gegebenenfalls noch übliche Textilhilfsmittel oder/und Farbstoffe zugefügt werden können, und mit dieser Flotte das zu schützende Material imprägniert. Die beiden Wirkstoffkomponenten (A) und (B) können der Applikationsflotte selbstverständlich auch getrennt zugegeben werden.

Die zu schützenden Materialien, insbesondere Textilmaterialien, können z.B. durch heisse oder kalte wässrige Färbe-, Bleich-, Chromierungs- oder Nachbehandlungsbäder, die einen bestimmten Teil einer erfindungsgemässen Wirkstoffkombination enthalten, imprägniert werden, wobei verschiedene Textilausrüstungsverfahren, wie z.B. das Foulard- oder Ausziehverfahren, in Frage kommen.

Die Behandlung erfolgt zweckmässig bei Temperaturen von 10 bis 100°C, im Färbebad vorzugsweise bei etwa 60-100°C, im Nachbehandlungsoder Waschbad bei vorzugsweise 10 bis 70, insbesondere 20 bis 60°C.

Als zusätzliche Hilfsmittel können den Applikationsflotten z.B. Dispergatoren, Emulgatoren oder Tenside zugegeben werden, sofern nicht schon genügend davon durch das erfindungsgemässe Mittel eingebracht wird. Daneben kann die Flotte auch noch übliche Hilfsstoffe, wie wasserlösliche perborate, Polyphosphate, Carbonate, Silikate, optische Aufheller, Weichmacher, sauer reagierende Salze, wie Ammonium- oder Zinksilikonfluorid, oder gewisse organische Säuren, wie malsäure, Essigsäure oder besonders Ameisensäure,

ferner Antimikrobika und Appreturmittel, z.B. solche auf Kunstharzbasis oder Stärke, enthalten. Falls die Motten- und Käferechtausrüstung gemeinsam mit dem Färben des Materials (z.B. Wolle) durchgeführt wird, enthalten die Flotten auch noch die entsprechenden Farbstoffe und gegebenenfalls die dazu erforderlichen Hilfsmittel, z.B. Egalisiermittel.

Wässrige Applikationsflotten können z.B. Tenside, beispielsweise anionaktive Verbindungen, wie Seifen und andere Carboxylate (z.B. Alkalisalze höherer Fettsäuren), Abkömmlinge von Schwefel-Sauerstoffsäuren (z.B. Natriumsalz der Dodecylbenzolsulfonsäure, wasserlösliche Salze von Schwefelsäuremonoestern höhetmolekularer Alkohole oder ihrer Polyglykoläther, wie etwa lösliche 8alze von Dodecylalkoholsulfat oder von Dodecylalkoholpolyglykoläther-sulfat), Abkömmlinge von Phosphor-Sauerstoffsäuren (z.B. Phosphate), Abkömmlinge mit saurem (elektrophilem) Stickstoff in der hydrophilen Gruppe (z.B. Disulfinsalze), kationaktive Tenside, wie Amine und ihre Salze (z.B. Lauryl-diäthylentriamin), Oniumverbindungen, Aminoxide oder nichtionogene Tenside, wie Polyhydroxyverbindungen, Tenside auf Mono- oder Polysaccharidbasis, höhermolekulare Acetylenglykole, Polyglykoläther (z.B. Polyglykoläther höherer Fettalkohole, Polyglykoläther höhermolekular-alkylierter Phenole) enthalten.

Im Falle von nicht-wässriger Applikation (Lösungsmittelapplikation) kann ein entsprechender Teil eines erfindungsgemässen Mittels (Wirkstoffkombination) auch einem geeigneten Lösungsmittel zugegeben werden und mit der so erhaltenen Lösung kann das zu schützende Material imprägniert werden. Als Lösungsmittel kommen hierfür unter anderen Trichloräthylen, Methylenchlorid, Kohlenwasserstoffe, Propylenglykol, Methoxyäthanol, Aethoxyäthanol, Dimethylformamid in Frage, denen noch Verteilungsmittel (z.B. Emulgatoren, wie sulfiertes Ricinusöl, Fettalkoholsulfate usw.) und/oder andere Hilfsstoffe zugesetzt werden können. Die zu schützenden Materialien werden üblicherweise mit diesen Lösungen einfach imprägniert.

Die Ausrüstung der zu schützenden Materialien kann aber auch mit einem Trockenreinigungsprozess kombiniert werden. Ein entsprechender Teil eines erfindungsgemässen Mittels (Wirkstoffkombination) wird zu diesem Zweck im Reinigungsmittel (etwa niedere halogenierte Alkane, z.B. Trichloräthylen usw.) gelöst und der Reinigungsprozess wird wie üblich durchgeführt.

Ein Anteil eines erfindungsgemässen Mittels (Wirkstoffkombination) kann aber auch in leicht flüchtigen organischen Lösungsmitteln gelöst werden und diese Lösung kann dann auf das zu schützende Substrat aufgesprüht werden (Sprühapplikation). Für diese Applikationsart eignen sich vor allem wollhaltige Textilien, Pelze und Federn. Der Vorteil der Sprühapplikation besteht darin, dass wegen der Rückgewinnung der Lösungsmittel eine Belastung der Abwässer vermieden wird.

Im erfindungsgemässen Verfahren können die erfindungsgemässen Mittel auch in Kombination mit anderen gegen keratinfressende Insekten wirksamen Schutzmitteln angewendet werden, z.B. in Kombination mit Harnstoffderivaten, Benzimidazolen, aromatischen Sulfonamiden und phosphor- und Phosphonsäureestern.

Die jeweils eingesetzte Menge an erfindungsgemässem Mittel (an erfindungsgemässer Wirkstoffkombination), die in das jeweilige Applikationsbad bzw. das nichtwässrige Lösungsmittel eingebracht wird, hängt vom jeweiligen Substrat und der Applikationsmethode ab. Diese Menge wird üblicherweise jedoch so bemessen, dass nach den Aufziehen auf das jeweils zu schützende Material letzteres etwa l0 bis 2 000 ppm, vorzugsweise l00 bis 1 000 ppm der Wirksubstanzkombination, also an·Barbiturat + Pyrethroid (an Komponente (A) i (B))enthält, wobei die obere Grenze weitgehend durch Ueberlegungen ökonomischer Natur gegeben ist, während die untere Grenze von Kriterien wie angestrebte Breite und Dauerhaftigkeit der Schutzwirkung abhängt. Dies ergibt z.B. für das Ausziehverfahren bei einem Flottenverhältnis von 1:20 Konzentrationen von 0,001 bis 1 g Wirksubstanz/1 Behandlungsbad, je nach erreichbarem Ausziehgrad. Beim Foulardverfahren sind Konzentrationen bis 2 g Wirksubstanz pro Liter möglich.

In den nachfolgenden Herstellungsvorschriften und Beispielen sind, sofern nichts anderes angegeben ist, Teile Gewichtsteile und prozente Gewichtsprozente. Unter den Bezeichnungen "Permethrin" und "Cypermethrin" sind die vorstehend definierten Verbindungen der Formel (3) mit $Y'_1 = Y_4 = Y'_4 =$ Wasserstoff bzw. mit $Y'_1 =$ CN und $Y_4 = Y_4' =$ Wasserstoff zu verstehen.

Herstellungsvorschriften für einige 5-Phenylcarbamoylbarbitursäuren der Formel (1):

1. 13,7 g (0,06 Mol) 1,3-Dimethyl-5-äthoxycarbonylbarbitursäure und 16,9 g (0,06 Mol) 2,6-Dimethyl-4-(4-trifluormethylphenoxy)-anilin werden miteinander gut vermischt und unter Schutzgasatmosphäre (Stickstoff) 1 Stunde auf 160°C (Aussentemperatur) erhitzt, wobei Aethanol entweicht. Zur Vervollständigung der Reaktion wird der Kolbeninhalt weitere 10 Minuten auf 180°C (Aussentemperatur) erhitzt, anschliessend auf Raumtemperatur abgekühlt und 2 mal aus Toluol/Hexan (1:1) umkristallisiert. Man erhält 19,6 g 1,3-Dimethyl-5-(2,6-dimethyl-4-(4-trifluormethylphenoxy))-carbamoylbarbitursäure der Formel

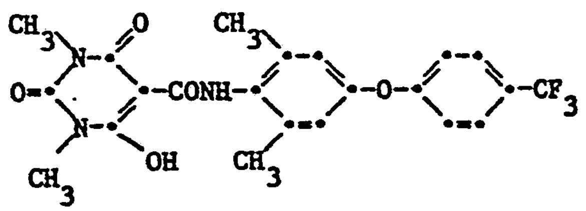 (101)

mit einem Schmelzpunkt von 189-191°C.

2. 9,1 g (0,04 Mol) 1,3-Dimethyl-5-äthoxycarbonylbarbitursäure und 11,8 g (0,04 Mol) 2-Isopropyl-4-(4-trifluormethylphenoxy)-anilin werden miteinander gut vermischt und unter Schutzgasatmosphäre (Stickstoff) 1 Stunde auf 160°C (Aussentemperatur) erhitzt, wobei Aethanol entweicht. Zur Vervollständigung der Reaktion wird der Kolbeninhalt weitere 10 Minuten auf 180°C (Aussentemperatur) erhitzt, anschliessend auf Raumtemperatur abgekühlt und 2 mal aus Benzin umkristallisiert. Man erhält 11,8 g 1,3-Dimethyl-5-[2-isopropyl-4-(4-trifluormethylphenoxy)]carbamoylbarbitursäure der Formel

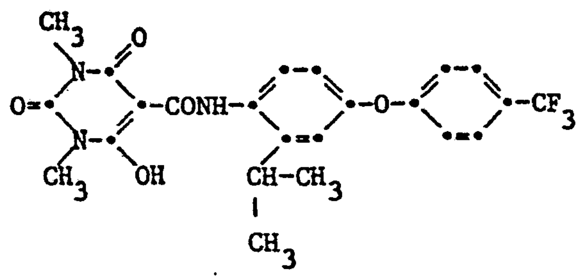 (102)

mit einem Schmelzpunkt von 158-159°C.

Die übrigen, von Formel (1) umfassten 5-Phenylcarbamoylbarbitursäuren können analog vorstehender Verfahrensweise erhalten werden. Alternativ können die Verbindungen der Formel (1) auch nach dem in der EP-A 7541 beschriebenen Verfahren erhalten werden, nämlich durch Umsetzung einer Barbitursäure der Formel

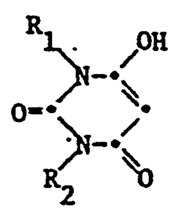

mit einem Isocyanat der Formel

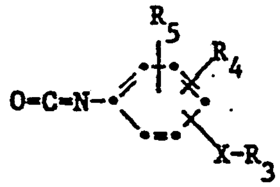

unter den in besagter Ep-A angegebenen Bedingungen. Bin Teil der von Formel (1) umfassten Verbindungen ist aus der Ep-A 7541 bekannt.

**Beispiel 1**

Von einer Mischung (1:1) aus der Verbindung der Formel (101) und permethrin wird eine 0,4%ige Stammlösung in Aethylenglykolmonomethyläther bereitet. Dann werden bei Zimmertemperatur wässrige Applikationsflotten hergestellt, die in 120 ml destilliertem Wasser 0,12 ml eines Netz- und Dispergiermittels, 0,6 ml Ameisensäure 1:10 und 0,2 ml, 0,1 ml bzw. 0,05 ml der 0,4%igen Stammlösung enthält. Dann werden jeweils 3 g schwere Wollflanell-Gewebestücke mit heissem Wasser durchnetzt und bei Zimmertemperatur eingegeben. Unter ständigem Umziehen der Wollmuster wird die Badtemperatur innerhalb 20 Minuten auf

98°C erhöht und 60 Minuten bei 98°C gehalten. Dann wird abgekühlt, die Wollmuster werden zweimal 3 Minuten mit destilliertem Wasser gespült, von Hand abgequetscht und an der Luft getrocknet. Die Wirkstoffkonzentration beträgt 250, 125 bzw. 60 ppm, berechnet auf das Wollgewicht.

Das so getrocknete Muster wird der Mottenechtheitsprüfung (Frassschutz gegen Kleidermotte Tineola bisselliella Hum.) gemäss der Vorschrift des schweizerischen Normenverbandes SNV 195901 sowie der Echtheitsprüfung gegen Larven des Pelzkäfers (Attagenus piceus 01.) und Teppichkäfers (Anthrenus vorax Wat.) gemäss SNV 195902 unterworfen.

Es werden jeweils Larven von Anthrenus vorax und 6 bis 7 Wochen alte Larven von Attagenus piceus zur Prüfung verwendet. Aus den behandelten Wollflanellmustern werden Stücke gleicher Grösse ausgeschnitten und 14 Tage lang bei konstanter Temperatur (28°C) und konstanter relativer Luftfeuchtigkeit (65%) dem Angriff (Frass) von je 15 Larven des entsprechenden Schädlings ausgesetzt. Die Beurteilung erfolgt einerseits nach dem relativen Gewichtsverlust des Prüflings und andererseits nach der Anzahl noch lebender Organismen.

Die geprüfte Wirkstoffmischung zeigt eine ausgezeichnete Wirkung gegen alle 3 verwendeten Schädlinge, und zwar bei allen verwendeten Konzentrationen.

Verwendet man eine Mischung (1:1) aus der Verbindung der Formel (101) und Cypermethrin oder dem Pyrethroid der Formel

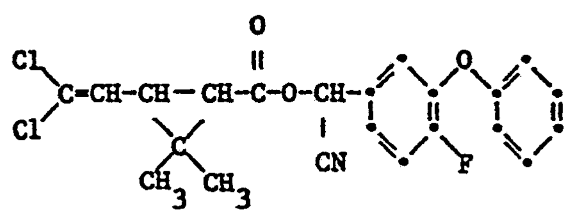

(201)

bzw. jeweils eine 1:1-Mischung der Verbindung der Formel (102) mit Permethrin, Cypermethrin oder der Verbindung der Formel (201) und verfährt wie oben beschrieben, so erhält man ebenfalls ein vollständig motten- und käferecht ausgerüstetes Gewebe.

**Beispiel 2**

Im Ausrüstungsverfahren gemäss Beispiel 1 kann als Pyrethroidkomponente (Komponente (B)) auch eine oder mehrere der folgenden Verbindungen eingesetzt werden:

3-(2;2-Dichlorvinyl)-2,2-dimethylcyclopropancarbonsäure-(3-phenoxy-α-vinylbenzyl)ester und -(3-phenoxy-α-methyläthinylbenzyl)ester,

3-(2-Methyl-propen(1)yl)-2,2-dimethylcyclopropancarbonsäure-(4-fluor-3-phenoxybenzyl)-ester und -(4-fluor-3-phenoxy-α-cyano-benzyl)ester,

3-(2,2-Dichlorvinyl)-2,2-dimethylcyclopropancarbonsäure-(4-fluor-3-phenoxybenzyl)ester und -(4-fluor-3-phenoxy-α-äthinylbenzyl)ester,

3-(2,2-Dibromvinyl)-2,2-dimethylcyclopropancarbonsäure-(4-fluor-3-phenoxybenzyl)ester, -(4-fluor-3-phenoxy-α-cyanobenzyl)ester und -(4-fluor-3-phenoxy-α-äthinyl-benzyl)ester,

3-(2-Methyl-propen(1)yl)-2,2-dimethylcyclopropancarbonsäure-(4-fluor-3-(4-fluorphenoxy)-α-cyanobenzyl)ester,

3-(2,2-Dichlorvinyl)-2,2-dimethylcyclopropancarbonsäure-(4-fluor-3-(3-fluorphenoxy)benzylester, -(4-fluor-3-(3-fluorphenoxy)-α-cyano-benzyl)ester, -(4-fluor-3-(4-fluorphenoxy)benzyl)ester und -(4-fluor-3-(4-fluorphenoxy)-α-cyanobenzyl)ester,

3-(2,2-Dibromvinyl)-2,2-dimethylcyclopropancarbonsäure-(4-fluor-3-(3-fluorphenoxy)benzyl)ester, -(4-fluor-3-(3-fluorphenoxy)-α-cyano-benzyl)-ester und -(4-fluor-3-(4-fluorphenoxy)-α-cyanobenzyl)ester,

3-(2-Chlor-2-(4-chlorphenyl)vinyl)-2,2-dimethylcyclopropancarbonsäure-(4-fluor-3-phenoxy-α-cyanobenzyl)ester,

3-(2,2-Dichlorvinyl)-2,2-dimethylcyclopropancarbonsäure-(2-[oder 3-]-fluor-3-(3-[oder 4-]fluorphenoxy)-α-cyanobenzyl)ester,

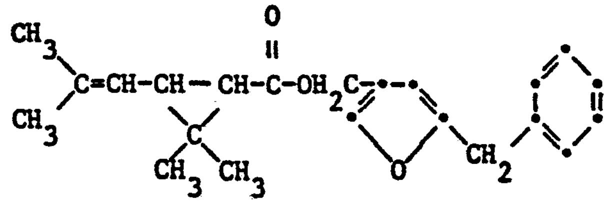 ;

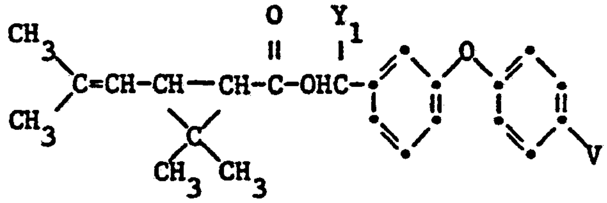 ,

worin V = H, Cl, Br, F, $CH_3$ oder $NO_2$ und

$Y_1$ = H, CN, $CH_3$, $C_2H_5$, i-$C_3H_7$, 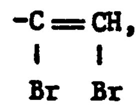, $-C\equiv CH$, $-C\equiv C-CH_3$,

$-CH=CH_2$,

$-CH=CH-CH_3$, $-CH_2-CH=CH_2$ oder $-CH_2-CH=CHCl$;

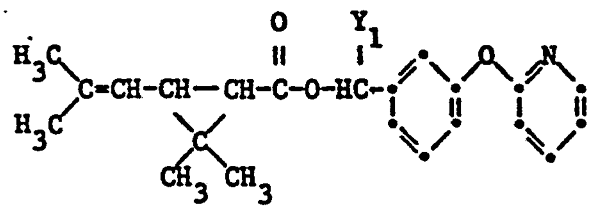 ,

worin $Y_1$ = H, CN oder $CH_3$;

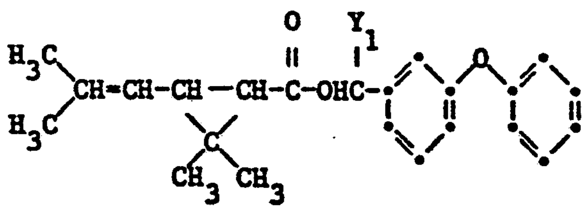

worin $Y_1$ = H, CN oder $-C\equiv CH$;

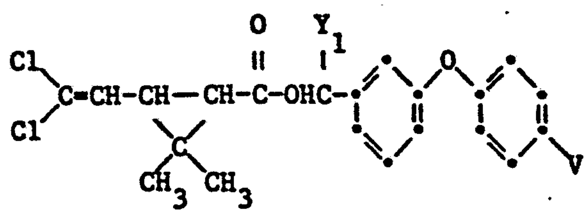 ,

worin V = H, Cl, Br, F, $CH_3$ oder $NO_2$ und

$Y_1$ = H, CN, $CH_3$, $C_2H_5$, i-$C_3H_7$, -C═CH, -C≡CH, -C≡C-$CH_3$, -CH=$CH_2$,
Br Br

-CH=CH-$CH_3$, -$CH_2$-CH=$CH_2$, -$CH_2$-CH=CHCl oder -C≡C-$C_6H_5$;

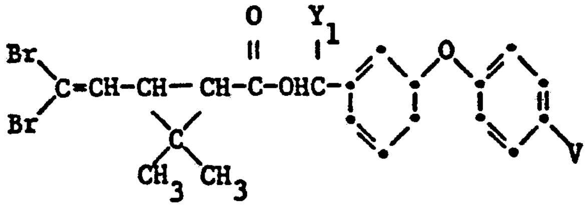
,

worin V = H, Cl, Br, F, $CH_3$ oder $NO_2$ und

$Y_1$ = H, CN, $CH_3$, $C_2H_5$, i-$C_3H_7$, -C = CH, -C≡CH, -C≡C-$CH_3$,
Br Br

-CH=$CH_2$,
-CH=CH-$CH_3$, -$CH_2$-CH=$CH_2$, -$CH_2$-CH-CHCl oder -C≡C-$C_6H_5$;

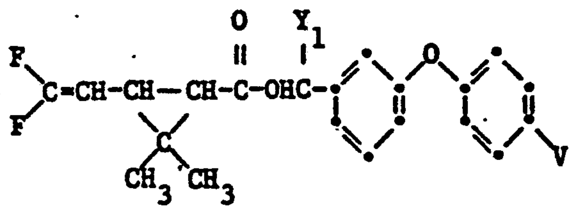
,

worin V = H, Cl, Br, F, $CH_3$ oder $NO_2$ und

$Y_1$ = H, CN, $CH_3$, $C_2H_5$, i-$C_3H_7$, -C = CH, -C≡CH, -C≡C-$CH_3$,
-CH=$CH_2$, Br Br

-CH=CH-$CH_3$, -$CH_2$-CH=$CH_2$, -$CH_2$-CH=CHCl oder -C≡C-$C_6H_5$;

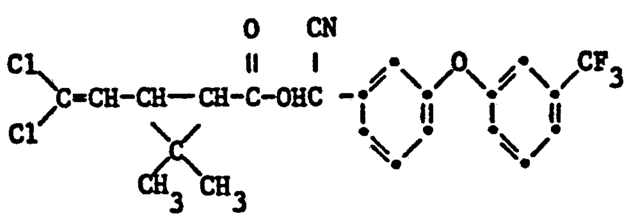
;

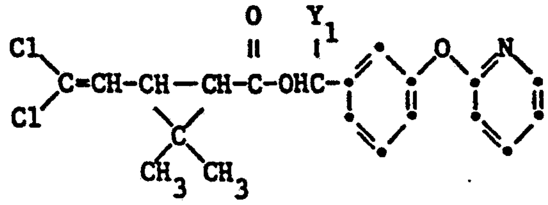 ,

worin $Y_1$ = H, CN oder $CH_3$;

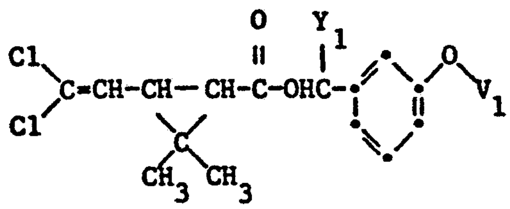 ,

worin $Y_1$ = H, CN oder $CH_3$ und
$V_1$ = $CH_2$-$CH$=$CH_2$, -$CH_2$-$C$≡$CH$, -$CH_2$-$CH$=$CH$-$CH_3$,

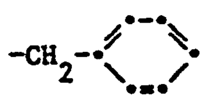 , 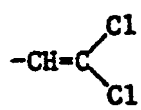 , 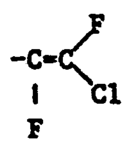 oder 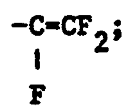 ;

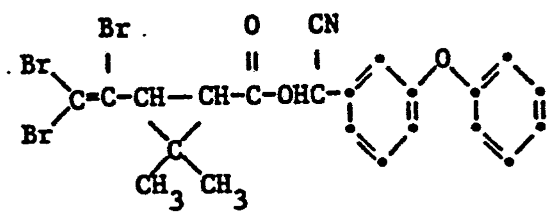 ;

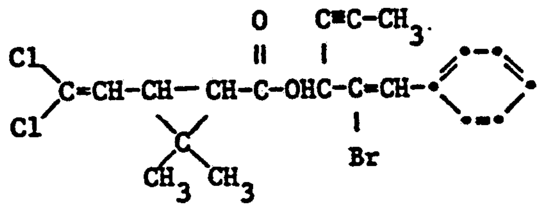

;

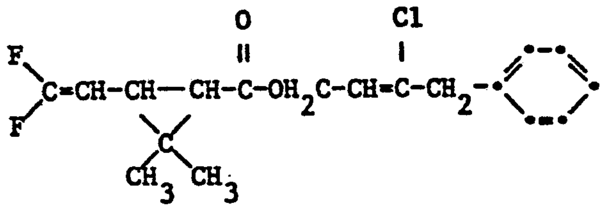

;

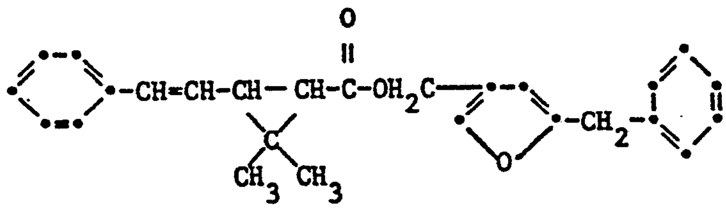

;

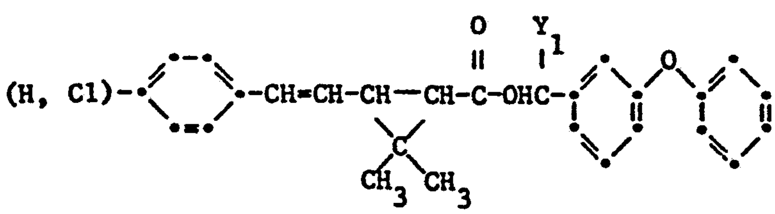

,

worin $Y_1 = H, CN, CH_3, C_2H_5, i-C_3H_7, -\underset{Br}{C} = \underset{Br}{CH}, -C\equiv CH, -C\equiv C-CH_3,$
$-CH=CH_2,$
$-CH=CH-CH_3, -CH_2-CH=CH_2$ oder $-CH_2-CH=CHCl;$

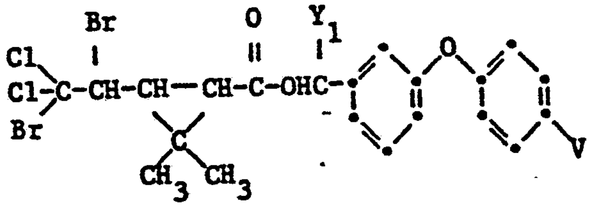
,

worin V = H, Cl, Br, F, $CH_3$ oder $NO_2$ und

$Y_1 = H, CN, CH_3, C_2H_5, i-C_3H_7, -\underset{Br}{C} = \underset{Br}{CH}, -C\equiv CH, -C\equiv C-CH_3,$
$-CH=CH_2,$
$-CH=CH-CH_3, -CH_2-CH=CH_2$ oder $-CH_2-CH=CHCl;$

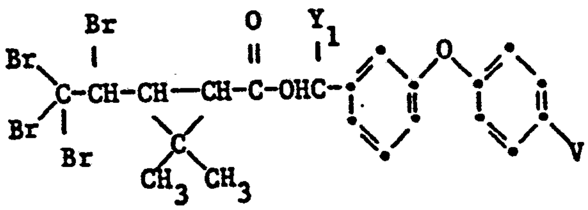
,

worin V = H, Cl, Br, F, $CH_3$ oder $NO_2$ und

$Y_1 = H, CN, CH_3, C_2H_5, i-C_3H_7, -\underset{Br}{C} = \underset{Br}{CH}, -C\equiv CH, -C\equiv C-CH_3,$

$-CH=CH-CH_3, -CH_2-CH=CH_2$ oder $-CH_2-CH=CHCl;$

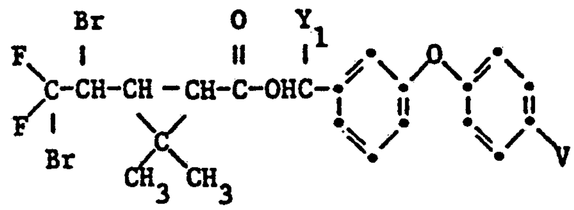

,

worin V = H, Cl, Br, F, $CH_3$ oder $NO_2$ und

$Y_1$ = H, CN, $CH_3$, $C_2H_5$, i-$C_3H_7$, $-\underset{Br}{C}=\underset{Br}{CH}$, $-C\equiv CH$, $-C\equiv C-CH_3$,

$-CH=CH-CH_3$, $-CH_2-CH=CH_2$ oder $-CH_2-CH=CHCl$;

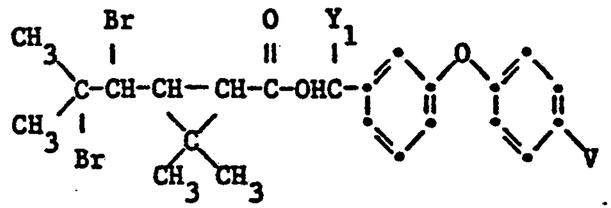

,

worin V = H, Cl, Br, F, $CH_3$ oder $NO_2$ und

$Y_1$ = H, CN, $CH_3$, $C_2H_5$, i-$C_3H_7$, $-\underset{Br}{C}=\underset{Br}{CH}$, $-C\equiv CH$, $-C\equiv C-CH_3$,

$-CH=CH-CH_3$, $-CH_2-CH=CH_2$ oder $-CH_2-CH=CHCl$;

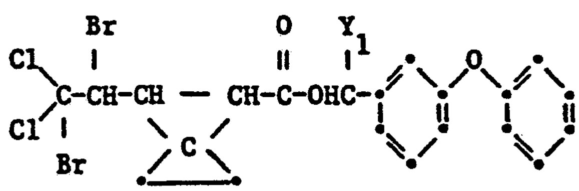

,

worin $Y_1$ = H oder CH;

worin $Y_1$ = H oder CN;

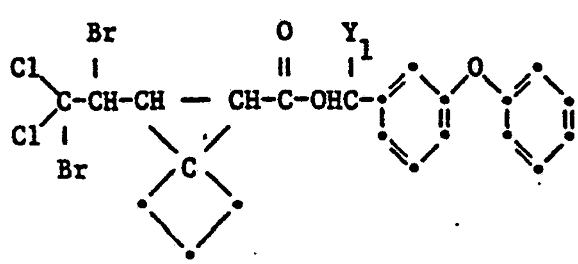

,

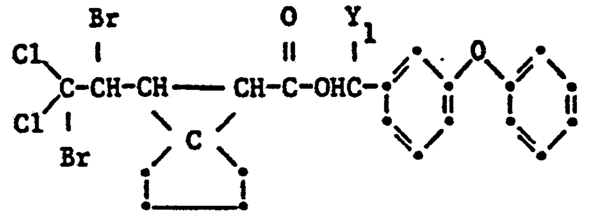,

worin $Y_1$ = H oder CN;

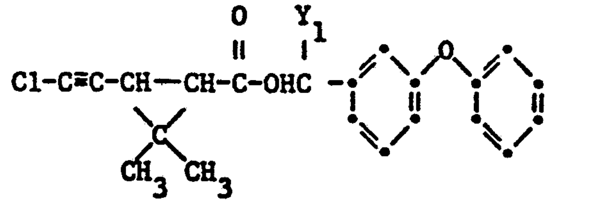,

worin $Y_1$ = H, CN oder $-CH{=}CH-CH_3$;

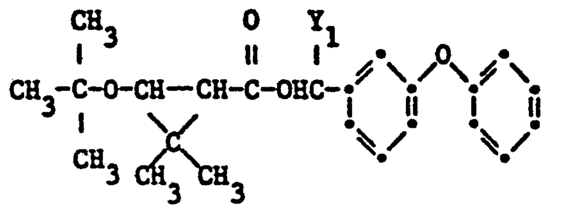,

worin $Y_1$ = H oder CN;

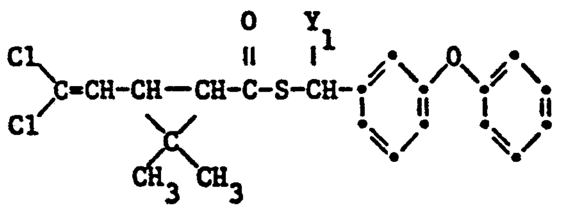,

worin $Y_1$ = H, $CH_3$ oder CN;

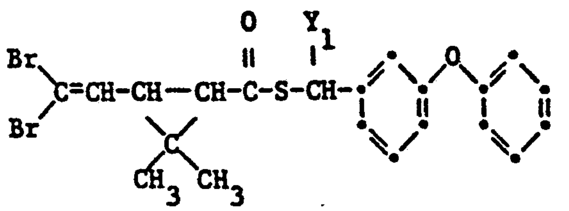,

worin $Y_1$ = H oder $CH_3$;

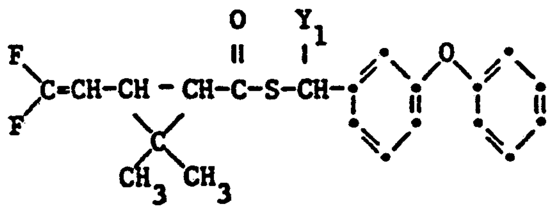

,

worin X = H oder $CH_3$;

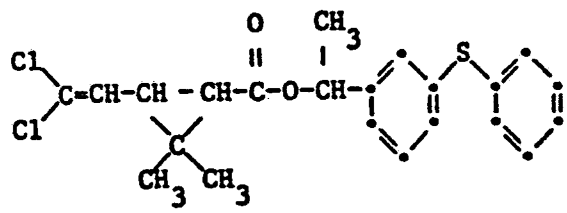

,

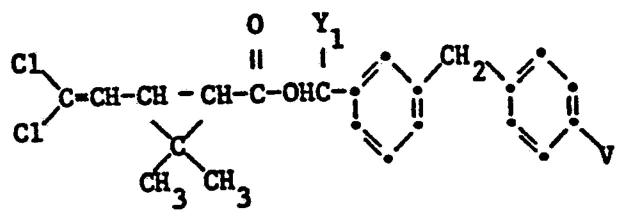

,

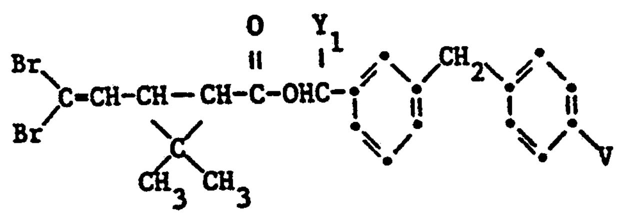

,

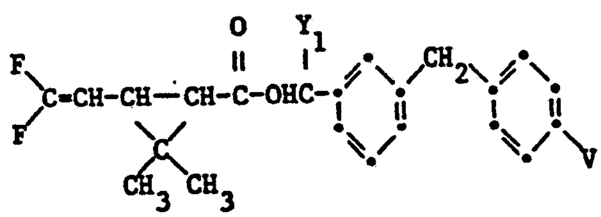

,

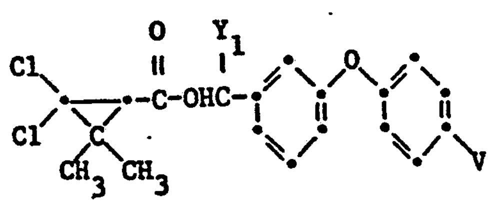

,

worin jeweils
V = H, Cl, Br, F, $CH_3$ oder $NO_2$ und

$-CH{=}CH_2$,

$-CH{=}CH{-}CH_3$, $-CH_2{-}CH{=}CH_2$, $-CH_2{-}CH{=}CHCl$ oder $-C{\equiv}C{-}C_6H_5$;

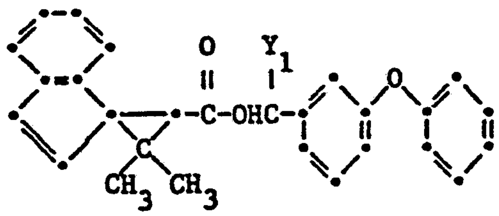

,

worin $Y_1$ = H, CN, $CH_3$ oder $-C{\equiv}C{-}CH_3$;

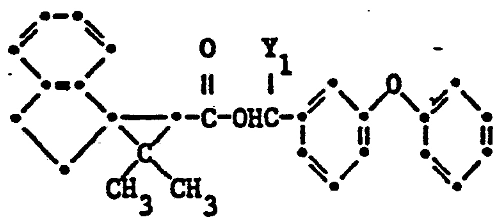

,

worin $Y_1$ = H, CN, $CH_3$ oder $-C{\equiv}C{-}CH_3$;

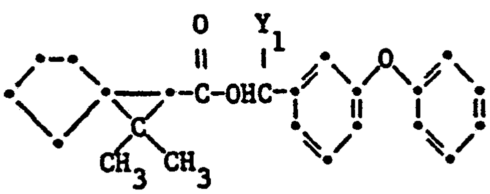

,

worin $Y_1$ = H, CN, $CH_3$ oder $-C{\equiv}C{-}CH_3$;

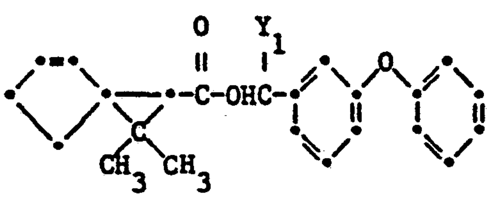

,

worin $Y_1$ = H, CN, $CH_3$ oder $-C{\equiv}C{-}CH_3$;

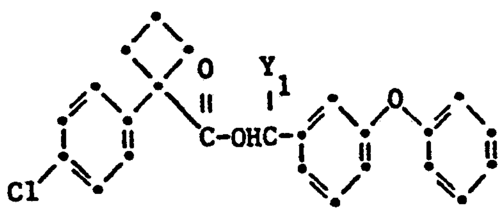

,

worin $Y_1$ = H, CN oder $-C{\equiv}CH$;

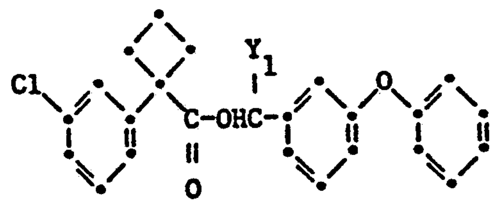
,

worin $Y_1$ = H oder CN;

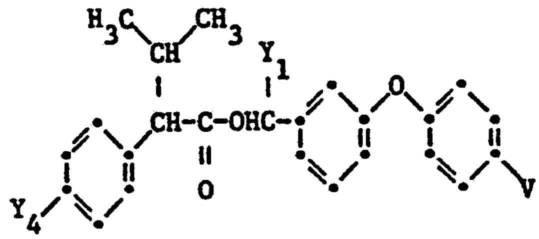
,

worin V = H, Cl, Br, F, $CH_3$ oder $NO_2$ und

$Y_1$ = H, CN, $CH_3$, $C_2H_5$, i-$C_3H_7$, $-C(Br)=CH(Br)$, $-C\equiv CH$, $-C\equiv C-CH_3$, $-CH=CH_2$, $-CH=CH-CH_3$, $-CH_2-CH=CH_2$, $-CH_2-CH=CHCl$ oder $-C\equiv C-C_6H_5$

und $Y_4$ = H, $CH_3$, Cl, $NO_2$, CN, $-OCH_3$, $-O-CH(CH_3)_2$, $-O-CH_2-C\equiv CH$

oder $-O-CH_2-CH=CH_2$;

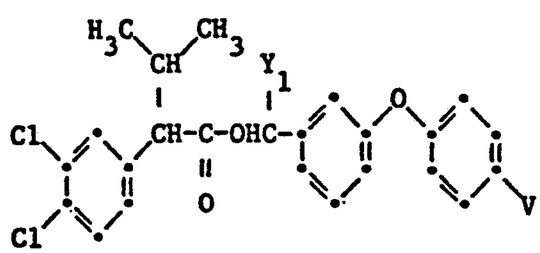
,

worin V = H, Cl, Br, F, $CH_3$ oder $NO_2$ und

$Y_1 = H, CN, CH_3, C_2H_5, i\text{-}C_3H_7, -\underset{Br}{C} = \underset{Br}{CH}, -C\equiv CH, -C\equiv C-CH_3,$
$-CH=CH_2,$
$-CH=CH-CH_3, -CH_2-CH=CH_2$ oder $-CH_2-CH=CHCl;$

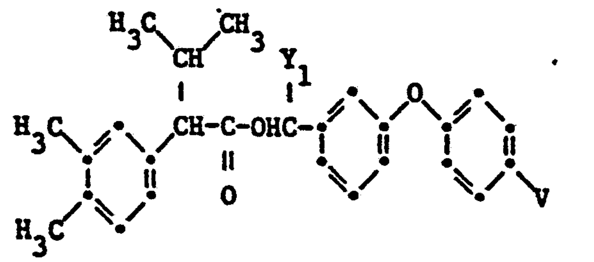
,

worin V = H, Cl, Br, F, $CH_3$ oder $NO_2$ und

$Y_1 = H, CN, CH_3, C_2H_5, i\text{-}C_3H_7, -\underset{Br}{C} = \underset{Br}{CH}, -C\equiv CH, -C\equiv C-CH_3,$
$-CH=CH_2,$
$-CH=CH-CH_3, -CH_2-CH=CH_2$ oder $-CH_2-CH=CHCl;$

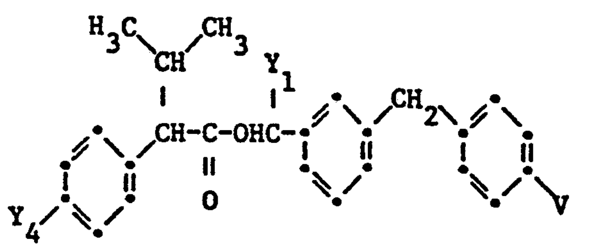
,

worin V = H, Cl, Br, F, $CH_3$ oder $NO_2$ und

$Y_1 = H, CN, CH_3, C_2H_5, i\text{-}C_3H_7, -\underset{Br}{C} = \underset{Br}{CH}, -C\equiv CH, -C\equiv C-CH_3,$
$-CH=CH_2,$
$-CH=CH-CH_3, -CH_2-CH=CH_2$ oder $-CH_2-CH=CHCl$

und $Y_4 = H, CH_3, Cl, NO_2, CN, -OCH_3, -O-CH\genfrac{}{}{0pt}{}{CH_3}{CH_3}, -OCH_2-C\equiv CH$

oder $-O-CH_2-CH=CH_2$;

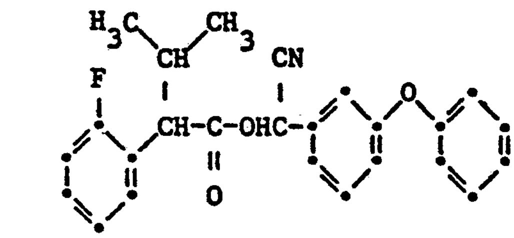

,

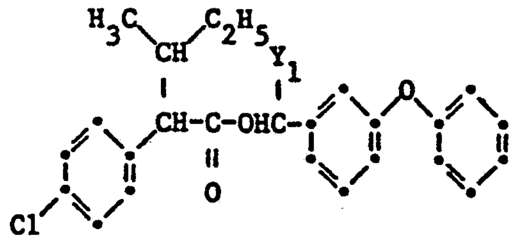

,

worin $Y_1$ = H oder CN;

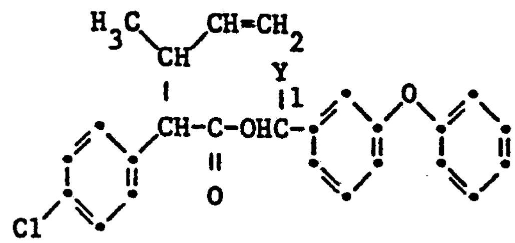

,

worin $Y_1$ = H oder CN;

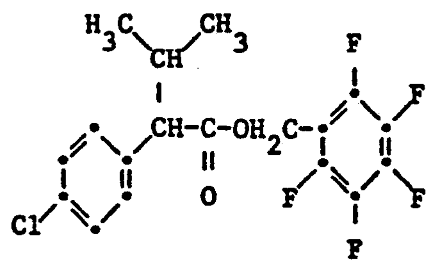

;

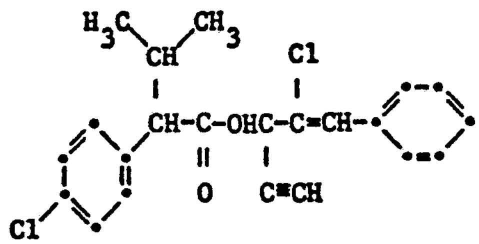 ;

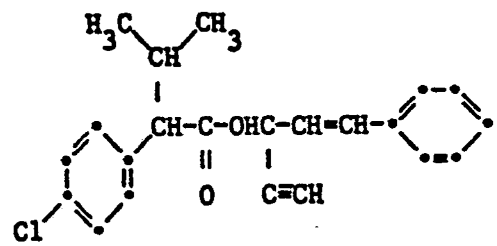 ;

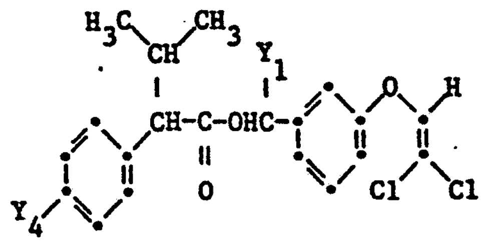 ,

worin $Y_1$ = H, CN, oder $CH_3$ und
$Y_4$ = H oder Cl;

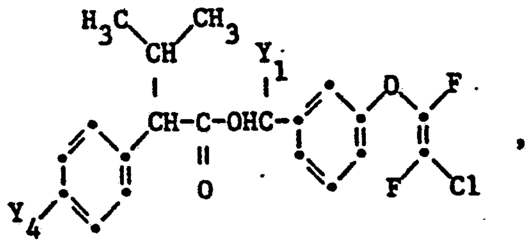

worin $Y_1$ = H, CN oder $CH_3$ und $Y_4$ = H oder Cl;

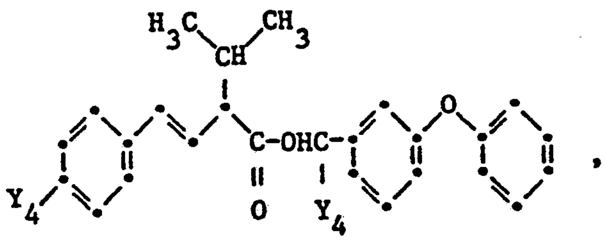

worin $Y_1$ = H, CN oder $CH_3$ und $Y_4$ = H oder $CH_3$;

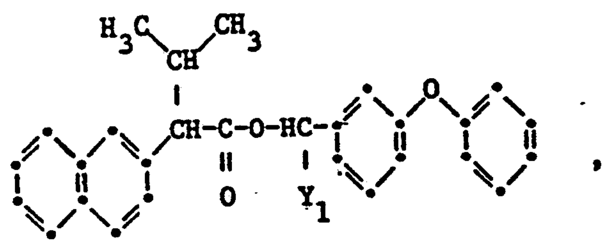

worin $Y_1$ = H oder CN;

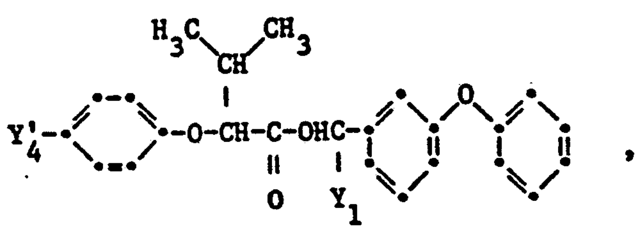

worin $Y_1$ = H oder CN und $Y'_4$ = Cl, $CH_3$ oder H;

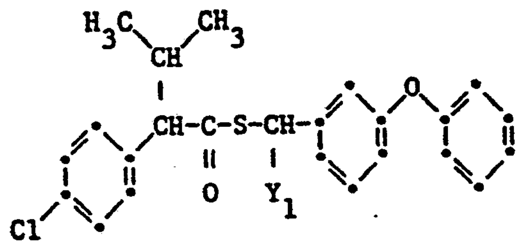
,

worin $Y_1$ = H, $CH_3$, -C≡CH oder CN;

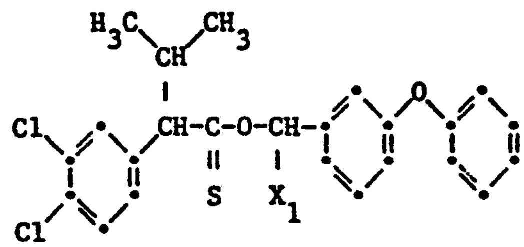
,

worin $Y_1$ = H oder CN;

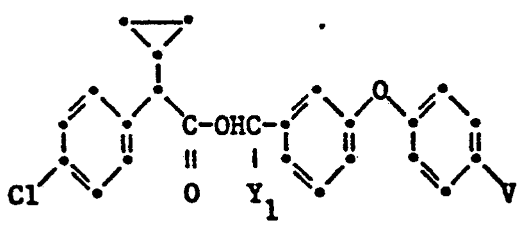
,

worin V = H, Cl, Br, F, $CH_3$ oder $NO_2$ und

$Y_1$ = H, CN, $CH_3$, $C_2H_5$, i-$C_3H_7$, -C(Br) = CH(Br), -C≡CH, -C≡C-$CH_3$, -CH=$CH_2$, -CH=CH-$CH_3$, -$CH_2$-CH=$CH_2$ oder -$CH_2$-CH=CHCl.

**Beispiel 3**

Im Ausrüstungsverfahren gemäss Beispiel 1 oder 2 kann als Barbituratkomponente (Komponente (A)) neben den bereits im Beispiel 1 genannten auch eine oder mehrere der in der nachfolgenden Tabelle 1 angeführten Verbindungen der allgemeinen Formel

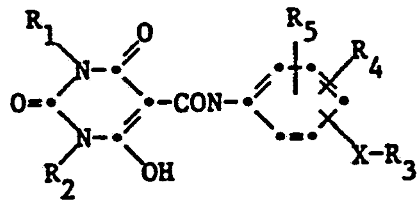

eingesetzt werden.

<u>Tabelle 1:</u>

| Verbindung Nr. | $R_1$ | $R_2$ | $R_4$ | $R_5$ | $X-R_3$ | Schmelzpunkt (°C) |
|---|---|---|---|---|---|---|
| 103 | 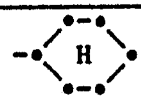 | $CH_3$ | $2-CH(CH_3)_2$ | H | 4-O-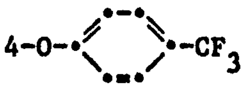-$CF_3$ | 157-158 |
| 104 | $CH_3$ | $CH_3$ | $2-CH_3$ | H | 4-O-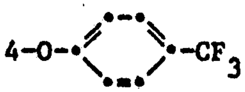-$CF_3$ | 163-165 |
| 105 | $CH_3$ | $CH_3$ | H | H | 4-O-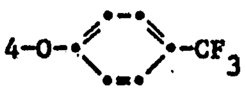-$CF_3$ | 192-194 |
| 106 | 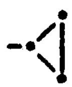 | $CH_3$ | $2-CH_3$ | $6-CH_3$ | 4-O-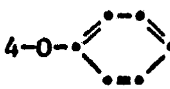-$CF_3$ | 147-149 |
| 107 | 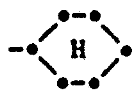 | $CH_3$ | $2-CH_3$ | $6-CH_3$ | 4-O-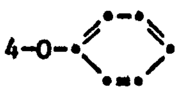-$CF_3$ | 100-102 |
| 108 | $CH_2CH=CH_2$ | $CH_3$ | $2-CH_3$ | $6-CH_3$ | 4-O-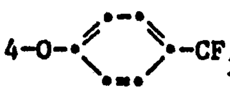-$CF_3$ | 139-140 |
| 109 | $CH_3$ | $CH_3$ | 3-Cl | H | $4-OCF_3$ | 160 |
| 110 | $C_2H_5$ | $C_2H_5$ | 3-Cl | H | $4-OCF_3$ | 134-135 |
| 111 | $C_2H_5$ | $CH_3$ | 2-Cl | H | $4-OCF_3$ | 119 |

Tabelle 1: (Fortsetzung)

| Verbindung Nr. | $R_1$ | $R_2$ | $R_4$ | $R_5$ | $X-R_3$ | Schmelzpunkt (°C) |
|---|---|---|---|---|---|---|
| 112 | $C_2H_5$ | $CH_3$ | 3-Cl | H | $4-OCF_2CHClF$ | 124 |
| 113 | $C_2H_5$ | $CH_3$ | H | H | $4-OCF_2CF_2H$ | 119 |
| 114 | $C_2H_5$ | $CH_3$ | H | H | $4-OCF_3$ | 118-120 |
| 115 | $C_2H_5$ | $CH_3$ | 3-Cl | H | $4-OCF_3$ | 130-132 |
| 116 | $C_2H_5$ | $CH_3$ | H | H | $3-OCF_2CHClF$ | 98-99 |
| 117 | $C_2H_5$ | $CH_3$ | 3-Cl | H | $4-SCF_2Cl$ | 138-140 |
| 118 | $C_2H_5$ | $CH_3$ | H | H | $4-SCF_3$ | 142 |
| 119 | $C_2H_5$ | $CH_3$ | H | $3,4-O-CF_2-O-CF_2-$ | | 98-100 |
| 120 | $C_2H_5$ | $CH_3$ | H | H | 4-O- 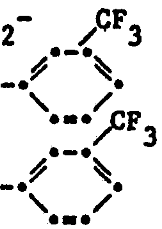 | 172-180 |
| 121 | $C_2H_5$ | $CH_3$ | $3-CF_3$ | H | 4-O- | 63-70 |
| 122 | $C_2H_5$ | $CH_3$ | 3-Cl | H | $4-O-CF_3$ | |
| 123 | $C_2H_5$ | $CH_3$ | 3-Cl | H | $4-S-CF_3$ | |
| 124 | $CH_3$ | $CH_3$ | 3-Cl | H | $4-SCF_3$ | |

Tabelle 1: (Fortsetzung)

| Verbindung Nr. | $R_1$ | $R_2$ | $R_4$ | $R_5$ | $X-R_3$ | Schmelzpunkt (°C) |
|---|---|---|---|---|---|---|
| 125 | $C_2H_5$ | $C_2H_5$ | 3-Cl | H | 4-$SCF_3$ | |
| 126 | $C_2H_5$ | $CH_3$ | 2-Cl | H | 4-$OCF_3$ | |
| 127 | $CH_3$ | $CH_3$ | 2-Cl | H | 4-$OCF_3$ | |
| 128 | $C_2H_5$ | $C_2H_5$ | 3-Cl | H | 4-$OCF_2CHClF$ | |
| 129 | $CH_3$ | $CH_3$ | 3-Cl | H | 4-$OCF_2CHClF$ | |
| 130 | $CH_3$ | $CH_3$ | H | H | 4-$OCF_3$ | |
| 131 | $CH_3$ | $CH_3$ | H | H | 4-$OCF_2CHF_2$ | |
| 132 | $CH_3$ | $C_2H_5$ | H | H | 4-$OCF_2CHF_2$ | |
| 133 | $CH_3$ | $CH_3$ | H | H | 3-$OCF_2CHClF$ | |
| 134 | $CH_3$ | $C_2H_5$ | 3-Cl | H | 4-$OCF_2CF_2Cl$ | |
| 135 | $CH_3$ | $CH_3$ | 3-Cl | H | 4-$SCF_2Cl$ | |
| 136 | $CH_3$ | $C_2H_5$ | H | H | 3-$SCF_3$ | |
| 137 | $CH_3$ | $CH_3$ | 2-$CH(CH_3)_2$ | H | 4-O— 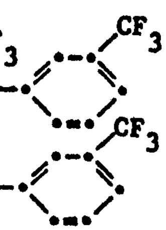 | 143-145 |
| 138 | $CH_3$ | $CH_3$ | H | H | 4-O— | 197-199 |
| 139 | $CH_3$ | $CH_3$ | H | 2,3-O-$CF_2$-O-$CF_2$- | | |

Tabelle 1: (Fortsetzung)

| Verbindung Nr. | $R_1$ | $R_2$ | $R_4$ | $R_5$ | $X-R_3$ | Schmelzpunkt (°C) |
|---|---|---|---|---|---|---|
| 140 | $CH_3$ | $CH_3$ | 5-Cl | H | 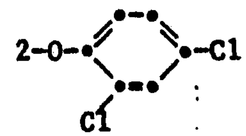 | 240-241 |
| 141 | $CH_3$ | $CH_3$ | H | H | 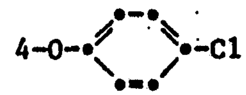 | 209-210 |
| 142 | $CH_3$ | $CH_3$ | 4-Cl | H | 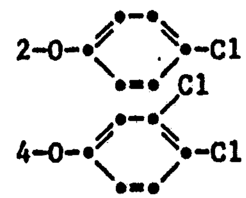 | 255-256 |
| 143 | $CH_3$ | $CH_3$ | 2-$CH_3$ | 6-$CH_3$ | | 210-212 |
| 144 | $CH_3$ | $CH_3$ | 3-Cl | H | 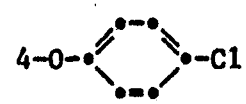 | 183-184 |
| 145 | 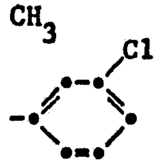 | $CH_3$ | 3-Cl | H | 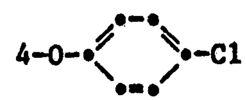 | 141-142 |
| 146 | $CH_3$ | $CH_3$ | 3-Cl | 4-Cl | 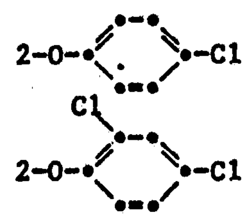 | 270-272 |
| 147 | $CH_3$ | $CH_3$ | H | H | | 215-217 |
| 148 | $CH_3$ | $CH_3$ | H | H | 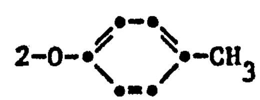 | 240-243 |

Tabelle 1: (Fortsetzung)

| Verbindung Nr. | $R_1$ | $R_2$ | $R_4$ | $R_5$ | $X-R_3$ | Schmelzpunkt (°C) |
|---|---|---|---|---|---|---|
| 149 | $CH_3$ | $CH_3$ | 4-Cl | H | 2-O- 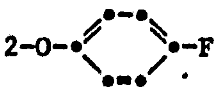 | 220-223 |
| 150 | $CH(CH_3)_2$ | $CH_3$ | 4-Cl | H | 2-O- 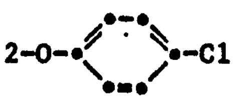 | 170-173 |
| 151 | $CH_3$ | $CH_3$ | H | H | 2-O- 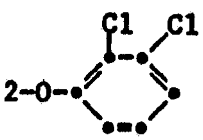 | 198-200 |
| 152 | $CH_3$ | $CH_3$ | 3-Cl | 4-Cl | 2-O- 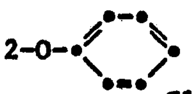 | 132-138 |
| 153 | $CH_3$ | $CH_3$ | 3-Cl | 4-Cl | 2-O- 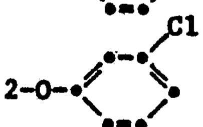 | 120-122 |
| 154 | $CH_3$ | $CH_3$ | $4-CH_3$ | H | 2-O- 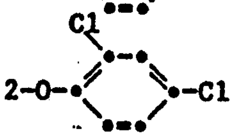 | 221-223 |
| 155 | $CH_3$ | $CH_3$ | 3-Cl | H | 2-O- 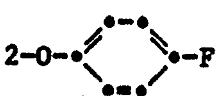 | > 250 |
| 156 | $CH_3$ | $CH_3$ | 4-Cl | H | 2-O- 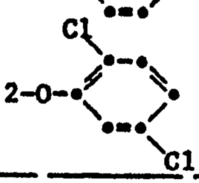 | 238-240 |

Tabelle 1: (Fortsetzung)

| Verbindung Nr. | $R_1$ | $R_2$ | $R_4$ | $R_5$ | $X-R_3$ | Schmelzpunkt (°C) |
|---|---|---|---|---|---|---|
| 157 | 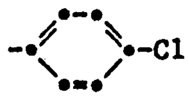 | $CH_3$ | 4-Cl | H | 2-O- 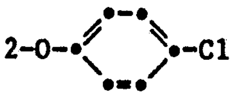 | > 280 |
| 158 | 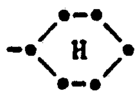 | $CH_3$ | 4-Cl | H | 2-O- 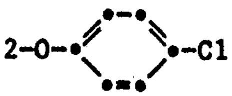 | 217-220 |
| 159 | $CH_3$ | $CH_3$ | 4-Cl | H | 2-O- 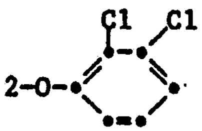 | 225-227 |
| 160 | 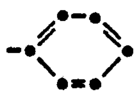 | $CH_3$ | 4-Cl | H | 2-O- 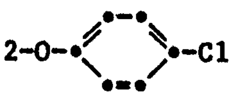 | 215-218 |
| 161 | $C_2H_5$ | $C_2H_5$ | 4-Cl | H | 2-O- 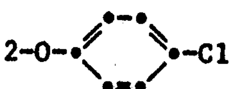 | 197-200 |
| 162 | $CH_3$ | $CH_3$ | 4-Cl | H | 2-O- 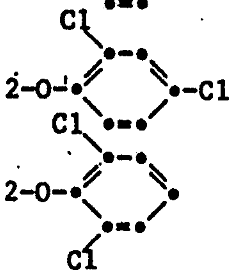 | 235-237 |
| 163 | $CH_3$ | $CH_3$ | 4-Cl | H | 2-O- | 262-264 |
| 164 | $CH_3$ | $CH_3$ | 4-Cl | H | 2-O- 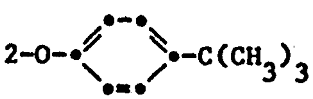 | 230-232 |

<u>Tabelle 1:</u> (Fortsetzung)

| Verbindung Nr. | $R_1$ | $R_2$ | $R_4$ | $R_5$ | $X-R_3$ | Schmelzpunkt (°C) |
|---|---|---|---|---|---|---|
| 165 | $-CH_2-CH=CH_2$ | $-CH_2-CH=CH_2$ | 4-Cl | H | 2-O- 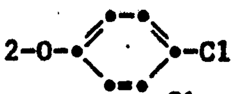 | 182-185 |
| 166 | $CH_3$ | $CH_3$ | 4-Cl | H | 2-O- 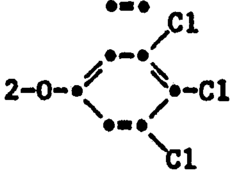 | 268-270 |
| 167 | $CH_3$ | $CH_3$ | $2-C_2H_5$ | 6- 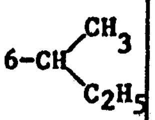 | 4-O- 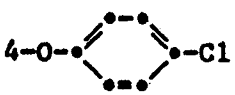 | 124-126 |
| 168 | $CH_3$ | $CH_3$ | H | H | 4-O- 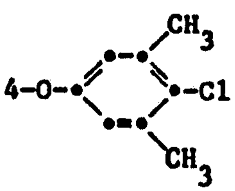 | 234-235 |
| 169 | $CH_3$ | $CH_3$ | $2-CH_3$ | $6-CH_3$ | 4-O- 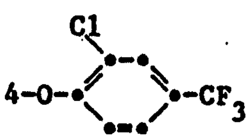 | 172-173 |
| 170 | $CH_3$ | $CH_3$ | $2-OCH_3$ | H | 3-O- 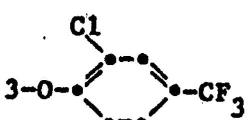 | 188-189 |
| 171 | 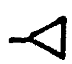 | $CH_3$ | H | H | 2-O- 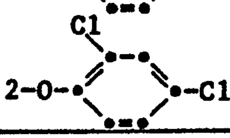 | 202-203 |

**Beispiel 4**

Von einer Mischung (1:1) aus der Verbindung der Formel (101) und Permethrin wird eine 0,4%ige Stammlösung in Aethylenglykolmonomethyläther bereitet. 12,5 ml der jeweiligen Stammlösung werden mit Aethylenglykolmonomethyläther, welcher 0,65 g/l eines Netz- und Dispergiermittels enthält, auf 50 ml verdünnt (= Lösung Nr. 2). 25 ml von Lösung Nr. 2 werden erneut mit Aethylenglykolmonomethyläther, welche 0,5 g/l eines Netz- und Dispergiermittels enthält, auf 50 ml verdünnt (= Lösung Nr. 3).

Von den Lösungen Nr. 1, 2 und 3 werden je 3 ml in Kristallisierschalen geleert und je eine geköderte Rondelle aus Wollflanell 3 Sekunden darin benetzt. Die feuchten Rondellen werden anschliessend zwischen Aluminiumfolien foulardiert, und zwar derart, dass die abgequetschten Rondellen je 50% Flotte aufgenommen haben. Die Konzentration an Wirkstoff auf den Rondellen beträgt dann der Reihe nach 500 ppm, 250 ppm und 125 ppm.

Die feuchten Rondellen werden an der Luft getrocknet und den gleichen biologischen Prüfungen unterworfen, wie in Beispiel 1 beschrieben.

Die geprüfte Wirkstoffmischung zeigt eine ausgezeichnete Wirkung gegen alle 3 Schädlinge, und zwar bei allen verwendeten Konzentrationen.

Verwendet man eine Mischung (1:1) ausder Verbindung der Formel (101) und Cypermethin oder der Verbindung der Formel (201) oder eine andere in Beispiel 1 angegebene Wirkstoffkombination oder eine sich aus den Beispielen 2 und 3 ergebende erfindungsgemässe Wirkstoffkombination und verfährt wie oben beschrieben, so erhält man ebenfalls ein vollständig motten- und käferecht ausgerüstetes Gewebe.

**Beispiel 5**

Es wird eine 10%ige Lösung ei-ner Mischung (1:1) aus der Verbindung der Formel (101) und Permethrin in Aethylenglykolmonomethyläther hergestellt. Ein Volumenteil dieser Lösung wird mit 200 Volumenteilen eines zur Trockenreinigung geeigneten Lösungsmittels, z.B. einer passenden Benzinfraktion oder Perchloräthylen, verdünnt. Gewünschtenfalls können noch reinigungsfördernde Zusätze beigefügt werden. Wollartikel werden nun wie üblich in dieser Reinigungsflüssigkeit behandelt und anschliessend auf einen Lösungsmittelgehalt von ca. 100% des Wollgewichtes abgeschleudert. Sie zeigen nach dem Trocknen eine gute Schutzwirkung gegen die oben genannten keratinfressenden Schädlinge.

**Beispiel 6**

Es wird jeweils eine 0,5%ige Lösung einer Mischung (1:1) aus der Verbindung der Formel (102) und Permethrin in Methylenchlorid, Trichloräthylen oder einer tiefsiedenen Benzinfraktion angesetzt. Ein Wollartikel wird mit Hilfe einer üblichen Sprüheinrichtung mit dieser Lösung besprüht, sodass 2 x 15 g/m$^2$ an Wirkstofflösung appliziert werden. Bei einem Ausnützungseffekt des Aerosols von 30% befinden sich dann etwa 400 ppm an Wirkstoffkombination auf dem Material. Das so ausgerüstete Wollgewebe zeigt gute Schutzwirkung gegen die oben genannten keratinfressenden Schädlinge.

Verwendet man eine Mischung (1:1) aus der Verbindung der Formel (101) umd Cypermethrin oder der Verbindung der Formel (201) oder eine andere in Beispiel 1 angegebene Wirkstoffkombination oder eine sich aus den Beispielen 2 und 3 ergebende erfindungsgemässe Wirkstoffkombination und verfährt wie in den Beispielen 5 und 6 beschrieben, so erhält man ebenfalls gegen den Befall durch Keratinschädlinge geschütztes Gewebe.

Die erfindungsgemässen Wirkstoffkombinationen können, wenn sie nicht direkt als solche zu Applikationsbädern zugesetzt werden, auch durch Zusatz von verschiedenen Trägern, Lösungsmitteln und/oder Hilfsmitteln formuliert werden. Besonders worteilhafte Formulierungen, die lagerstabil sind, erhält man, wenn man die beiden erfindungsgemässen Wirkstoffkomponenten in der in der EP-A 74 335 angegebenen Weise formuliert, d.h. eine Formulierung herstellt, die neben den beiden Komponenten (A) und (B) noch ein oder mehrere aliphatische(s) oder cycloaliphatische(s) Amin(e) oder/und Amid(e) und deren Derivat(e) und gegebenenfalls ein oder mehrere organische(s) Lösungsmittel, Wasser, ein(en) odermehrere Tensid(e) und/oder Emulgator(en) bzw. Dispergator(en) und/oder eine oder mehrere aliphatische Carbonsäure(n) enthält. Die erfindungsgemässen Wirkstoffkombinationen können also wie in den Beispielen 1-25 der genannten EP-A 74 335 beschrieben formuliert werden. Als Beispiele sollen die beiden folgenden Formulierungen dienen:

**Beispiel 7**

5,3 Teile Permethrin,
8,0 Teile 1-Hydroxyäthyl-2-oleyl-imidazolin,
7,0 Teile mit 6-7 Mol Aethylenoxid äthoxyliertes Talgfettamin,
73,7 Teile Diäthylenglykoläthyläther und
1,0 Teile Milchsäure, racemisch,

werden gemischt. Zu dieser Mischung werden unter ständigem Rühren bei 45-55°C 5,0 Teile der Verbindung der Formel (102) gegeben, bis eine homogene Formulierung entsteht. Diese so erhaltene Mottenschutzformulierung ist lagerstabil, mit Wasser mischbar und sie führt bei der Applikation auf Keratimmaterial zu ausgezeichneten Mottenund Käferechtausrüstungen.

**Beispiel 8**

Die Formulierung folgender Zusammensetzung wird analog Beispiel 7 erhalten:.
5,5 Teile Permethrin,
5,0 Teile der Verbindung der Formel (101)
20 0 Teile mit 6-7 Mol Aethylenoxid äthoxyliertes Talgfettamin,
5,0 Teile eines Blockpolymeren aus Propylenglykol und Aethylenoxid (mittleres Molekulargewicht: 4900, 80% hydrophobe Gruppen, 20% hydrophile Gruppen; HLB = C),
7,0 Teile Rizinuspolyglykoläther
3,0 Teile Alkylphosphorsäurepartialester
54,5 Teile Isophoron (= 3,5,5-Trimethyl-2-cyclohexen-1-on).

**Beispiel 9**

Die Formulierung folgender Zusammensetzung wird analog Beispiel 7 erhalten:
5,5 Teile Permethrin
5,0 Teile der Verbindumg der Formel (101)
45,0 Teile der Verbindung der Formel

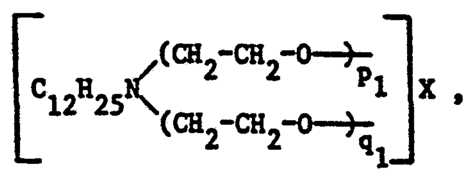

worin $p_1$ i $q_1$ = 8 und X einem sauren Phosphorsäurerest bedeutet,
20,0 Teile eimes Blockpolymerem aus Propylenglykol und Aethylenoxid (mittleres Molekulargewicht: 6350; 50% hydrophobe und 50% hydrophile Gruppem; HLB = 15),
12,5 Teile Nonylphenoläthersulfat, Na-8alz, mit 40 Mol Aethylenoxid äthoxyliert,
7,0 Teile Aethylpolyglykol,
5,0 Teile Polyäthylenglykol 300.

Setzt man in den Formulierungen der Beispiele 7-9 an 8telle von Permethrin Cypermethrin oder die Verbindung der Formel (201) oder an Stelle von Permethrin und Verbindung der Formel (101) bzw. (102) eine andere in Beispiel 1 angegebene Wirkstoffkombination oder eine sich aus den Beispielen 2 und 3 ergebende erfindungsgemässe Wirkstoffkombination ein, so kommt man ebenfalls zu vorteilhaften lagerstabilen Formulierungen, die zum Ausrüsten von Keratinmaterial gegen den Befall durch Keratinschädlinge verwendet werden können.

**Beispiel 10**

Färbung und gleichzeitige Motten- und Käferechtausrüstung:
Auf einem Färbeapparat wird ein Stück Wollgewebe in 600 g einer Färbeflotte, die aus
1,5 g der Formulierung gemäss Beispiel 7, 8 oder 9,
30,3 g Glaubersalz,

24,0 g Schwefelsäure conc.,
3,0 g eines roten Farbstoffes der Formel

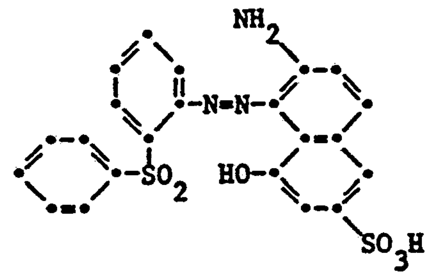

und

541,5 g entmineralisiertem Wasser
besteht, während 5 Minuten bei 40°C vorgenetzt (Flottenverhältnis 1:20). Die Flotte wird anschliessend innerhalb 45 Minuten auf ca. 98°C erhitzt. Nach einer einstündigen Behandlungsdauer bei dieser Temperatur wird das Wollgewebe gespült und getrocknet. Der Farbstoff und die beiden in der jeweiligen Formulierung enthaltenen Wirkstoffe sind auf das Gewebe aufgezogen. Das rotgefärbte Wollgewebe ist nach dieser einbadigen Behandlung vollständig gegen Motten- und Käferlarvenfrass geschützt. Dies wird durch Echtheitsptüfung gemäss SNV-Norm 195901 und 195902 festgestellt.

**Beispiel 11**

Anwendung im Nachbehandlungsbad:
Auf einem Färbeapparat wird ein Stück Wollgewebe in 400 g einer Nachbehandlungsflotte, die aus
1 g der Formulierung gemäss Beispiel 7, 8 oder 9,
4 g Ameisensäure 85% und
395 g entmineralisiertem Wasser
besteht, während 5 Minuten bei 30°C vorgenetzt (Flottenverhältis 1:20). Die Flotte wird dann innerhalb von 20 Minuten auf 45°C erwärmt. Nach einer 30 Minuten dauernden Behandlung bei dieser Temperatur, wobei das Wollgewebe unter ständiger Bewegung gchalten wird, wird letzteres in kaltem Wasser gut gespült und getrocknet. Das so behandelte Wollgewebe ist gegen die Larven von Wollschädlingen vollständig geschützt.

**Patentansprüche**

1. Mittel zum Schützen von Keratinmaterial gegen den Befall durch Keratinschädlinge, dadurch gekennzeichnet, dass es
(A) ein oder mehrere 5-Phenylcarbamoylbarbiturat(e) der Formel

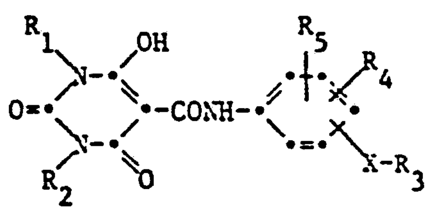

(1)

sowie deren tautomere Formen und Salze,
worin
$R_1$ und $R_2$ unabhängig voneinander $C_1$-$C_4$-Alkyl, $C_3$-$C_6$-Cycloalkyl, $C_2$-$C_4$-Alkenyl, Benzyl, Phenyl oder mit 1 bis 3 Substituenten aus der Gruppe Halogen, $C_1$-$C_4$-Alkyl, $C_1$-$C_4$-Alkoxy und Nitro substituiertes Phenyl,
$R_3$ $C_1$-$C_4$ -Halogenalkyl, Phenyl oder durch 1-3 Substituenten aus der Gruppe $C_1$-$C_4$-Halogenalkyl, Nitro, Halogen und $C_1$-$C_4$-Alkyl substituiertes Phenyl,
$R_4$ und $R_5$ unabhängig voneinander Wasserstoff, Halogen, $C_1$-$C_4$-Halogenalkyl, $C_1$-$C_4$-Alkyl, $C_2$-$C_4$-Alkenyl, $C_3$-$C_6$-Cycloalkyl, Methoxy oder Nitro und
X Sauerstoff oder Schwefel bedeuten, wobei X-$R_3$ gemeinsam mit $R_4$ oder $R_5$ in ortho-Stellung auch für die Gruppe -O-$CF_2$-O-$CF_2$- stehen kann,

und
(B) ein oder mehrere synthetische(s) Pyrethroid(e) der allgemeinen Formel

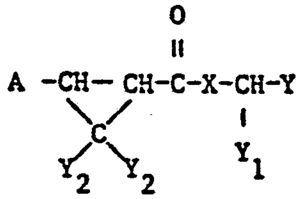

(2),

worin

A für $Br_2C=CBr-$, (H, Cl)- 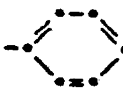 -CH=CH-, Cl-C≡C-, Cl- 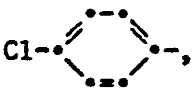 -,

$(CH_3)_3C-O-$, 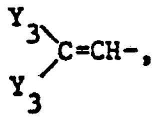 worin $Y_3$ für Cl, Br, $CF_3$, F oder $C_1$-$C_4$-Alkyl steht,

$CH_2=CH-CH_2-O-$ oder 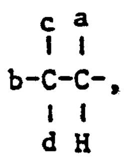 worin a, b, c und d unabhängig voneinander für Cl, Br oder F stehen, wobei c und d auch für Methyl stehen können,
X Sauerstoff oder Schwefel, $Y_1$ Wasserstoff, CN, $CH_3$, $C_2H_5$, i-$C_3H_7$,

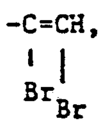 -C≡CH, $-C≡C-CH_3$, $-C≡C-C_6H_5$, $-CH=CH-CH_3$, $-CH_2-CH=CH_2$,

$-CH=CH_2$ oder $-CH_2-CH=CHCl$,
$Y_2$ Methyl oder beide $Y_2$ zusammen die Ergänzung zu einem Cyclopropan-, Cyclobutan- oder Cyclopentanring und

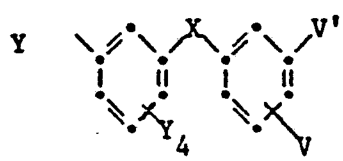

,

worin $Y_4$ für Wasserstoff oder Fluor und V für Wasserstoff, Cl, Br, F, $CH_3$ oder $NO_2$ und V' für Wasserstoff stehen, wobei V' für den Fall dass V Wasserstoff bedeutet, auch für $CF_3$ stehen kann, und X wie oben definiert ist; ferner Y

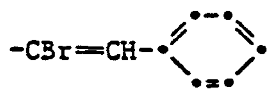 , 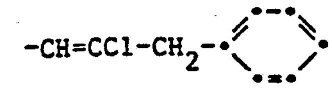 ,

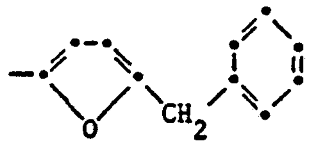 , 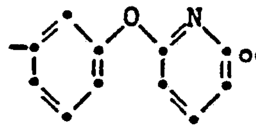 oder 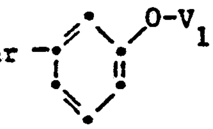 ,

bedeuten,
worin $V_1$ für $-CH_2-CH=CH_2$, $-CH_2-C\equiv CH$, $-CH_2-CH=CH-CH_3$

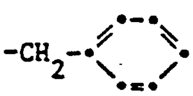 , 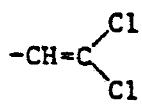 , -CF=CFCl oder $-CF=CF_2$ steht,

$-CF=CFCl$ oder $-CF=CF_2$ steht,
oder der allgemeinen Formel

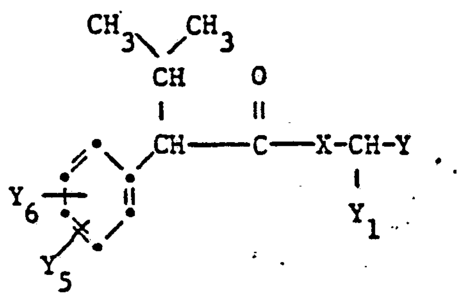 (2a),

worin X, Y und $Y_1$ wie in Formel (2) definiert sind, $Y_5$ Wasserstoff, $CH_3$, Cl, $NO_2$, $OCH_3$, $OCH(CH_3)_2$, $-OCH_2\equiv CH_2$ oder $OCH_2CH=CH_2$ und $Y_6$ Wasserstoff, $CH_3$, Cl, Br oder F oder $Y_5$ und $Y_6$ in ortho-Stellung gemeinsam die Ergänzung zu einem ankondensierten Benzolring bedeuten, enthält.

2. Mittel nach Anspruch 1, dadurch gekennzeichnet, dass es als Komponente (A) eine Verbindung der Formel (1) enthält, worin $R_1$ und $R_2$ unabhängig voneinander $C_1$-$C_4$-Alkyl oder $C_2$-$C_4$-Alkenyl, $R_3$ $C_1$-$C_4$-Halogenalkyl oder durch $C_1$-$C_4$-Halogenalkyl substituiertes Phenyl, $R_4$ und $R_5$ unabhängig voneinander Wasserstoff, Halogen, $C_1$-$C_4$-Halogenalkyl, Methoxy oder Nitro und für den Fall, dass $X$-$R_3$ für 4-Trifluormethylphenoxy steht, zusätzlich in 2- und/oder 6-Stellung $C_1$-$C_4$-Alkyl, $C_2$-$C_4$-Alkenyl oder Cyclopropyl, und X Sauerstoff oder Schwefel bedeuten, wobei $X$-$R_3$ gemeinsam mit $R_4$ oder $R_5$ in ortho-Stellung auch für die Gruppe $-O-CF_2-O-CF_2-$ stehen kann.

3. Mittel nach Anspruch 1 oder 2, dadurch gekennzeichnet, dass es als Komponente (A) eine Verbindung der Formel (1) enthält, worin X Sauerstoff, $R_1$ und $R_2$ $C_1$-$C_4$-Alkyl, insbesondere Methyl oder Aethyl bedeuten und der Substituent $-X-R_3$ in ortho- oder para-Stellung steht.

4. Mittel nach Anspruch 1 oder 2, dadurch gekennzeichnet, dass es als Komponente (A) eine Verbindung der Formel (1) enthält, worin $R_4$ und $R_5$ in 2- und 6-Stellung stehen und Wasserstoff oder $C_1$-$C_4$-Alkyl bedeuten und $-X-R_3$ für 4-Trifluormethylphenoxy steht.

5. Mittel nach einem der Ansprüche 1 bis 3, dadurch gekennzeichnet, dass es als Komponente (A) eine Verbindung der Formel (1) enthält, worin X Sauerstoff und $R_3$ $C_1$-$C_4$-Halogenalkyl bedeuten.

6. Mittel nach Anspruch 1 oder 3, dadurch gekennzeichnet, dass es als Komponente (A) eine Verbindung der Formel (1) enthält, worin X Sauerstoff und $R_3$ einen mit 1 bis 3, vorzugsweise 1 oder 2, Substituenten aus der Gruppe $C_1$-$C_4$ -Halogenalkyl, Halogen oder $C_1$-$C_4$-Alkyl substituierten Phenylrest bedeuten.

7. Mittel nach Anspruch 1, dadurch gekennzeichnet, dass es als Komponente (B) ein Pyrethroid der Formel (2) enthält, worin A einen Rest der Formel

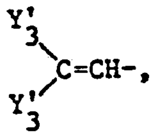

worin $Y'_3$ für Br, Cl oder $CH_3$ steht, X Sauerstoff und Y

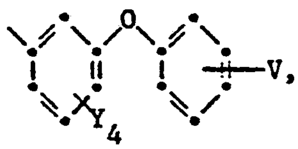

bedeuten, und $Y_2$ für $CH_3$ und $Y_1$ für Wasserstoff, CN, $CH_3$, $-CH{=}CH_2$, $-C{\equiv}CH$ oder $-C{\equiv}C-CH_3$ steht.

8. Mittel nach einem der Ansprüche 1 bis 7 dadurch gekennzeichnet, dass es neben den Komponenten (A) und (B) noch übliche Träger und/ oder Formulierungshilfsmittel, beispielsweise organische Lösungsmittel, Wasser, Säuren, Basen, Netz-, Dispergier- und/oder Emulgiermittel enthält.

9. Mittel nach Anspruch 8, dadurch gekennzeichnet, dass es neben den Komponenten (A) und (B) ein oder mehrere aliphatische(s) oder cycloaliphatische(s) Amin(e) oder/und Amid(e) und deren Derivat(e) und gegebenenfalfs ein oder mehrere organische(s) Lösungsmittel, Wasser, ein oder mehrere Tensid(e) und/oder Emulgator(en) bzw. Dispergator(en), oder/und eine oder mehrere aliphatische Carbonsäuren enthält.

10.Verfahren zum Schützen bzw. Ausrüsten von Keratinmaterial, insbesondere von Wolle, Pelzen, Federn und Fellen, vor bzw. gegen den Befall durch Keratinschädlinge, z.B. durch Motten- und Käferlarven, dadurch gekennzeichnet, dass man das zu schützende Material mit einer Wirkstoffkombination bestehend aus einer oder mehreren 5-Phenylcarbamoylbarbiturat(en) der Formel (1) bzw. deren tautomeren Formen und Salzen und einem oder mehreren synthetischen Pyrethroid(en) der Formel (2) oder (2a) nach Anspruch 1 behandelt.

11. Verfahren nach Anspruch 10, dadurch gekennzeichnet, dass man Verbindungen der Formel (1), worin X Sauerstoff, $R_1$ und $R_2$ $C_1$-$C_4$-Alkyl, insbesondere Methyl oder Aethyl bedeuten und der Substituent $-X-R_3$ in ortho- oder para-Stellung sceht, und synthetische Pyrethroide der Formel (2) oder (2a), insbesondere der Formel (2), einsetzt, vor allem solche, worin A einen Rest der Formel

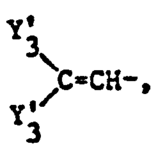

worin $Y'_3$ für Br, Cl oder $CH_3$ steht, X Sauerstoff und Y

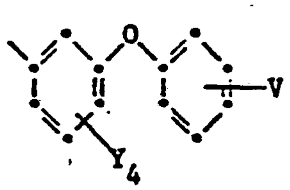

bedeuten, und $Y_2$ für $CH_3$ und $Y_1$ für Wasserstoff, CN, $CH_3$, $-CH{=}CH_2$, $-C{\equiv}CH$ oder $-C{\equiv}C-CH_3$ steht.

12. Verfahren nach Anspruch 10,dadurch gekennzeichnet, dass man ein Mittel einsetzt, das neben der Wirksroffkombination noch übliche Träger und/oder Formulierungshilfsmittel enthält.

13. Verfahren nach einem der Ansprüche 10 bis 12 dadurch gekennzeichnet, dass man das zu schützende Material, insbesondere wollfaserhalcige Textilien, mit einer wässrigen Flotte, die eine in Anspruch 12 definierte Wirkstoffkombination enthält, behandelt, wobei die wässrige Flotte gegebenenfalls noch übliche

Textilhilfsmittel, und/oder Farbstoffe enthalten kann, oder das zu schützende Material mit einer organischen Reinigungsflüssigkeit behandelt, die eine in Anspruch 12 definierte Wirkstoffkombination enthält, oder das zu schützende Material mit einem organischen Lösungsmittel besprüht, das eine in Anspruch 12 definierte Wirkstoffkombination enthält.

14. Verfahren nach einem der Ansprüche 10 bis 13 dadurch gekennzeichnet, dass man auf die zu schützenden Materialien die Wirkstoffkombination in einer Menge von 10 bis 2'000 ppm, vorzugsweise 100 bis 1'000 ppm, bezogen auf das zu schützende Material, aufbringt.

15. Das nach einem der Ansprüche 10 bis 14 ausgerüstete Keratinmaterial, vorzugsweise wollhaltige Textilien, Pelze und Felle, enthaltend 10 bis 2'000 ppm, vorzugsweise 100 bis 1'000 ppm, einer im Anspruch 12 definiercen Wirkstoffkombination.

16. Verwendung der in Anspruch 1 oder 2 definierten Mittel zum Schützen von Keratinmaterial, insbesondere von Wolltextilien, gegen den Befall durch Keratinschädlinge.

**Claims**

1. A composition for protecting keratinous material from attack by pests that feed on keratin, which composition contains

(A) one or more 5-phenylcarbamoylbarbiturates of the formula

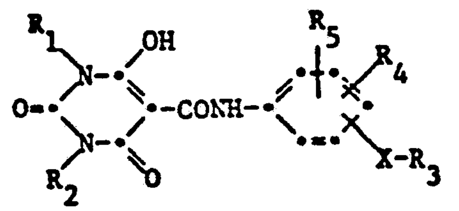

(1)

or tautomeric forms or salts thereof, in which formula each of $R_1$ and $R_2$ independently of the other is $C_1$-$C_4$alkyl, $C_3$-$C_6$cycloalkyl, $C_2$-$C_4$-alkenyl, benzyl, phenyl or phenyl which is substituted by 1 to 3 substituents selected from the group consisting of halogen, $C_1$-$C_4$alkyl, $C_1$-$C_4$alkoxy and nitro,

$R_3$ is $C_1$-$C_4$haloalkyl, phenyl or phenyl which is substituted by 1-3 substituents selected from the group consisting of $C_1$-$C_4$haloalkyl, nitro, halogen and $C_1$-$C_4$alkyl, each of $R_4$ and $R_5$ independently of the other is hydrogen, halogen, $C_1$-$C_4$haloalkyl, $C_1$-$C_4$alkyl, $C_2$-$C_4$alkenyl, $C_3$-$C_6$cycloalkyl, methoxy or nitro and X is oxygen or sulfur, and X-$R_3$, together with $R_4$ or $R_5$ in the ortho-position, may also be the group $-O-CF_2-O-CF_2-$, and

(B) one or more synthetic pyrethroids of the general formula

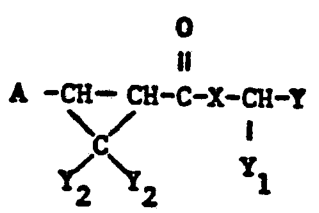

(2)

in which A is $Br_2C=CBr-$, (H, Cl)-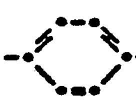-CH=CH-, Cl-C≡C-, Cl-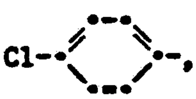-,

$(CH_3)_3C-O-$, 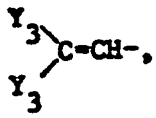

in which $Y_3$ is Cl, Br, $CF_3$, F or $C_1$-$C_4$alkyl, or A is $CH_2=CH-CH_2-O-$ or

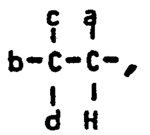

in which each of a, b, c and d independently is Cl, Br or F, and c and d may also be methyl, X is oxygen or sulfur, $Y_1$ is hydrogen, CN, $CH_3$, $C_2H_5$, i-$C_3H_7$,

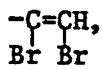

$-C\equiv CH$, $-C\equiv C-CH_3$, $-C\equiv C-C_6H_5$, $-CH=CH-CH_3$, $-CH_2-CH=CH_2$, $-CH=CH_2$ or $-CH_2-CH=CHCl$, $Y_2$ is methyl or the two $Y_2$s together complete a cyclopropane, cyclobutane or cyclopentane ring and Y is

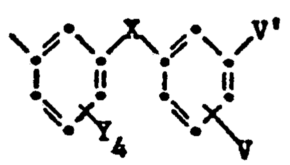 ,

in which $Y_4$ is hydrogen or fluorine and V is hydrogen, Cl, Br, F, $CH_3$ or $NO_2$ and V' is hydrogen, with the proviso that V' may also be $CF_3$ if V is hydrogen, and X is as defined above; and Y is also

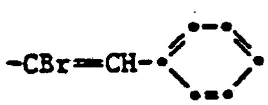 , 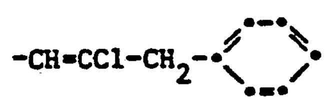 ,

Fl

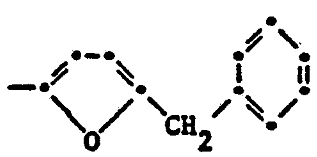 , 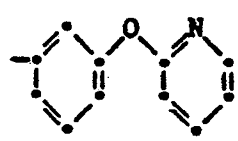 or 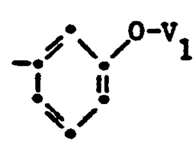 ,

in which $V_1$ is $-CH_2-CH=CH_2$, $-CH_2-C\equiv CH$, $-CH_2-CH=CH-CH_3$,

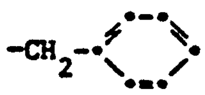 , 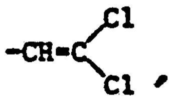 ,

$-CF=CFCl$ or $-CF=CF_2$; or of the general formula

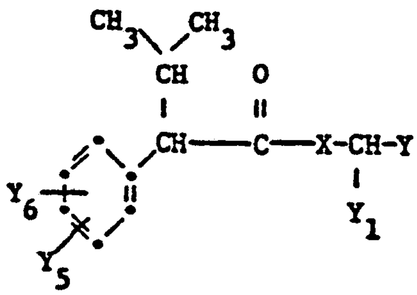

(2a)

in wich X, Y and $Y_1$ are as defined for formula (2), $Y_5$ is hydrogen, $CH_3$, Cl, $NO_2$, $OCH_3$, $OCH(CH_3)_2$, $-OCH_2C\equiv CH$ or $-OCH_2CH=CH_2$; and $Y_6$ is hydrogen, $CH_3$, Cl, Br or F, or $Y_5$ and $Y_6$ in the ortho-position together complete a fused benzene ring.

2. A composition according to claim 1, wherein component (A) is a compound of the formula (1) in which each of $R_1$ and $R_2$ independently of the other is $C_1$-$C_4$alkyl or $C_2$-$C_4$alkenyl, $R_3$ is $C_1$-$C_4$haloalkyl or phenyl which is substituted by $C_1$-$C_4$haloalkyl, each of $R_4$ and $R_5$ independently of the other is hydrogen, halogen, $C_1$-$C_4$haloalkyl, methoxy or nitro and, if $X$-$R_3$ is 4-trifluoromethylphenoxy, $R_4$ and $R_5$ are additionally $C_1$-$C_4$alkyl, $C_2$-$C_4$alkenyl or cyclopropyl in the 2-and/or 6-positions, and X is oxygen or sulfur, and $X$-$R_3$, together with $R_4$ or $R_5$ in the ortho-position, may also be the group $-O-CF_2-O-CF_2-$.

3. A composition according to either of claims 1 or 2, wherein component (A) is a compound of the formula (1) in which X is oxygen, $R_1$ and $R_2$ are $C_1$-$C_4$alkyl, in particular methyl or ethyl, and the substituent $-X-R_3$ is in the ortho- or para-position.

4. A composition according to either of claims 1 or 2, wherein component (A) is a compound of the formula (1) in which $R_4$ and $R_5$ are in the 2- and 6-positions and are hydrogen or $C_1$-$C_4$alkyl and $-X-R_3$ is 4-trifluoromethylphenoxy.

5. A composition according to any one of claims 1 to 3, wherein component (A) is a compound of the formula (1) in which X is oxygen and $R_3$ is $C_1$-$C_4$haloalkyl.

6. A composition according to either of claims 1 or 3, wherein component (A) is a compound of the formula (1) in which X is oxygen and $R_3$ is a phenyl radical which is substituted by 1 to 3, preferably 1 or 2, substituents selected from the group consisting of $C_1$-$C_4$halo-alkyl, halogen and $C_1$-$C_4$alkyl.

7. A composition according to claim 1, wherein component (B) is a pyrethroid of the formula (2) in which A is a radical of the formula

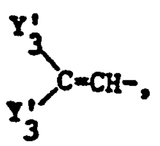

in which $Y'_3$ is Br, Cl or $CH_3$, X is oxygen and Y is

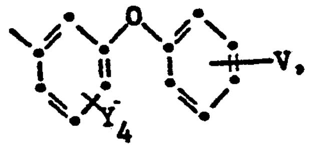

and $Y_2$ is ch$_3$ and $Y_1$ is hydrogen, CN, $CH_3$, $-CH=CH_2$, $-C\equiv CH$ or $-CH\equiv C-CH_3$.

8. A composition according to any one of claims 1 to 7, which, in addition to the components (A) and (8), also contains customary carriers and/or formulation auxiliaries, for example organic solvents, water, acids, bases, wetting agents, dispersing agents and/or emulsifiers.

9. A composition according to claim 8, which, in addition to the components (A) and (B), also contains one or more aliphatic or cycloaliphatic amines and/or amides and derivatives thereof and, if appropriate, one or more organic solvents, water, one or more surfactants and/or emulsifiers or dispersing agents and/or one or more aliphatic carboxylic acids.

10. A process for providing keratinous material, in particular wool, furs, feathers and hides, with a protective finish against attack by pests that feed on keratin, for example attack by moth and beetle larvae, which process comprises treating the material to be protected with an active ingredient combination consisting of one or more 5-phenylcarbamoylbarbiturates of the formula (1), or tautomeric forms or salts thereof, and one or more synthetic pyrethroids of the formula (2) or (2a) according to claim 1.

11. A process according to claim 10, which comprises the use of compounds of the formula (1) in which X is oxygen, $R_1$ and $R_2$ are $C_1$-$C_4$alkyl, in particular methyl or ethyl, and the substituent $-X-R_3$ is in the ortho- or para-position, and synthetic pyrethroids of the formula (2) or (2a), in particular of the formula (2), especially those in which A is a radical of the formula

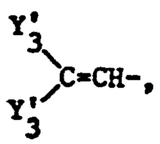

in which $Y'_3$ is Br, Cl or $CH_3$, X is oxygen and Y is

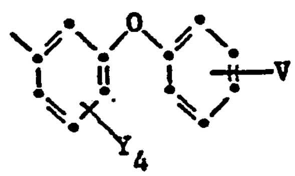

and $Y_2$ is $CH_3$ and $Y_1$ is hydrogen, CN, $CH_3$, $-CH=CH_2$, $-C\equiv CH$ or $-C\equiv C-CH_3$.

12. A process according to claim 10, which comprises the use of a composition which, in addition to the combination of active ingredients, also contains customary carriers and/or formulation auxiliaries.

13. A process according to any one of claims 10 to 12, wherein the material to be protected, in particular textiles made of or containing woollen fibres, is treated with an aqueous liquor containing an active ingredient combination as defined in claim 12, which aqueous liquor may also contain customary textile auxiliaries and/or dyes, or the material to be protected is treated with an organic cleaning fluid containing an active ingredient combination as defined in claim 12, or the material to be protected is sprayed with an organic solvent containing an active ingredient combination as defined in claim 12.

14. A process according to any one of claims 10 to 13, wherein the active ingredient combination is applied to the materials to be protected in an amount of 10 to 2,000 ppm, preferably 100 to 1,000 ppm, based on the material to be protected.

15. The keratinous material finished according to any one of claims 10 to 14, preferably wool-containing textiles, furs and hides, containing 10 to 2000 ppm, preferably 100 to 1,000 ppm, of an active ingredient combination as defined in claim 12.

16. Use of a composition as defined in either of claims 1 or 2 for protecting keratinous material, in particular wool textiles, from attack by pests that feed on keratin.

**Revendications**

1. Produit pour la protection de matériau à base de kératine contre l'attaque de parasites de la kératine, caractérisé par le fait qu'il contient

(A) un ou plusieurs 5-phénylcarbamylbarbiturate(s) de formule

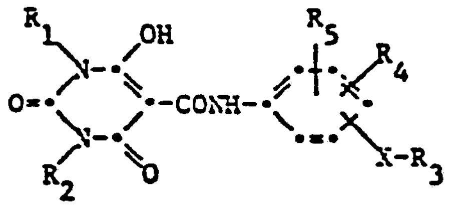

(1)

ainsi que leurs formes tautomères et leurs sels, dans laquelle formule $R_1$ et $R_2$ représentent, indépendamment l'un de l'autre, un radical alkyle en $C_1$-$C_4$, cycloalkyle en $C_3$-$C_6$, alcényle en $C_2$-$C_4$, benzyle, phényle ou phényle substitué par 1 à 3 substituants choisis parmi des atomes d'halogène, des groupes alkyle en $C_1$-$C_4$, alcoxy en $C_1$-$C_4$ et nitro;

$R_3$ représente un radical halogéno-alkyle en $C_1$-$C_4$, phényle ou phényle substitué par 1-3 substituants choisis parmi des atomes d'halogène, des groupes halogéno-alkyle en $C_1$-$C_4$, nitro et alkyle en $C_1$-$C_4$;

$R_4$ et $R_5$ représentent, indépendamment l'un de l'autre, un atome d'hydrogène, un atome d'halogène, un radical halogéno-alkyle en $C_1$-$C_4$, alkyle en $C_1$-$C_4$, alcényle en $C_2$-$C_4$, cycloalkyle en $C_3$-$C_6$, méthoxy ou nitro; et X représente un atome d'oxygène ou de soufre, X-$R_3$ pouvant également représenter, conjointement avec $R_4$ ou $R_5$ en position ortho, le groupe $-O-CF_2-O-CF_2-$;

et

(B) un ou plusieurs composé(s) synthétique(s) de type pyréthrine, de formule générale

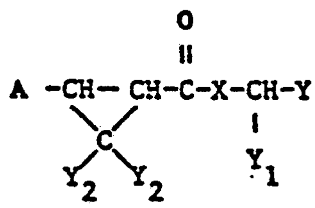

(2),

dans laquelle

A représente

$Br_2C=CBr-$, 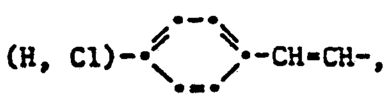 $Cl-C \equiv C-$, 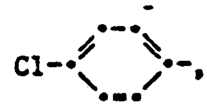

$(CH_3)_3C-O-$, 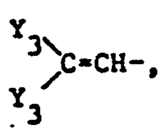

où $Y_3$ représente Cl, Br, $CF_3$, F ou un groupe alkyle en $C_1$-$C_4$,

$CH_2=CH-CH_2-O-$ ou

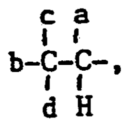,

où a, b, c et d représentent, indépendamment les uns des autres, Cl, Br ou F, c et d pouvant aussi représenter des groupes méthyle;

X est un atome d'oxygène ou de soufre;

$Y_1$ représante un atome d'hydrogène, CN, $CH_3$, $C_2H_5$, i-$C_3H_7$,

$-C=CH$, $-C\equiv CH$, $-C\equiv C-CH_3$, $-C\equiv C-C_6H_5$, $-CH=CH-CH_3$, $-CH_2-CH=CH_2$,
Br Br

$-CH=CH_2$ ou $-CH_2-CH=CHCl$,

$-CH=CH_2$ ou $-CH_2-CH=CHCl$,

$Y_2$ représente un radical méthyle, ou bien les deux radicaux $Y_2$ raprésentent ensemble le complément d'un noyau cyclopropane, cyclobutane ou cyclopentane; et

Y représente

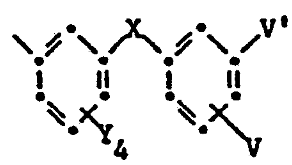 ,

où $Y_4$ représente un atome d'hydrogène ou de fluor et V représente un atome d'hydrogène, Cl, Br, F, $CH_3$ ou $NO_2$, et V' représente un atome d'hydrogène, V' pouvant également représenter $CF_3$ au cas où V est un atome d'hydrogène, et X est tel que défini plus haut; en outre Y représente

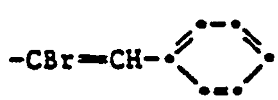 , 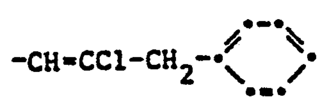 ,

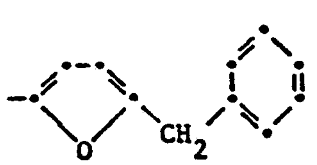 , 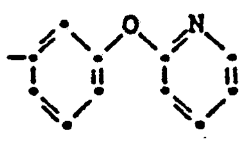 ou 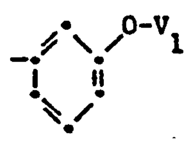 ,

où $V_1$ représente;
$-CH_2-CH=CH_2$, $-CH_2-C\equiv CH$, $-CH_2-CH=CH-CH_3$,

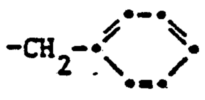 , 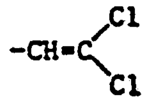 ,

$-CF=CGCl$ ou $-CF=CF_2$;
ou de formule générale

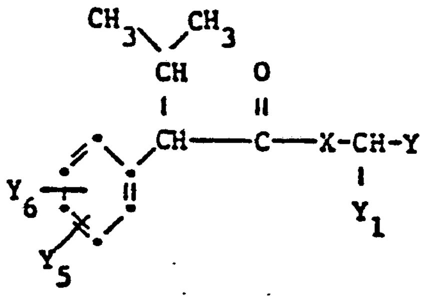 (2a),

dans lequelle X, Y et $Y_1$ sont tels que définis dans la formule (2); $Y_5$ représente un atome d'hydrogène, $CH_3$, Cl, $NO_2$, $OCH_3$, $OOH(CH_3)_2$, $-OCH_2C{\equiv}CH$ ou $-OCH_2CH{=}CH_2$; et $Y_6$ représente un atome d'hydrogène, $CH_3$, Cl, Br ou F; ou $Y_5$ et $Y_6$ en position ortho représentent ensemble le complément d'un noyau benzénique condensé.

2. Produit selon la revendication 1, caractérisé par le fait qu'il contient en tant que composant (A) un composé de formule (1) dans lequel $R_1$ et $R_2$ représentent, indépendamment l'un de l'autre, un radical alkyle en $C_1$-$C_4$ ou alcényle en $C_2$-$C_4$;

$R_3$ représente un radical halogéno-alkyle en $C_1$-$C_4$ ou un radical phényle substitué par un groupe halogéno-alkyle en $C_1$-$C_4$;

$R_4$ et $R_5$ représentent, indépendamment l'un de l'autre, des atomes d'hydrogène, d'halogène, ou des groupes halogéno-alkyle en $C_1$-$C_4$, méthoxy ou nitro, et au cas où X-$R_3$ représente le groupe 4-trifluorométhylphénoxy, ils représentent en outre en position 2 et/ou 6 des groupes alkyle en $C_1$-$C_4$, alcényle en $C_2$-$C_4$ ou cyclopropyle; et

X représente un atome d'oxygène ou de soufre, X-$R_3$ pouvant également représenter, conjointement avec $R_4$ ou $R_5$ en position ortho, le groupement $-O-CF_2-O-CF_2-$.

3. Produit selon la revendication 1 ou 2, caractérisé par le fait qu'il contient en tant que composant (A) un composé de formule (1) dans lequel X représente un atome d'oxygène; $R_1$ et $R_2$ représentent des groupes alkyle en $C_1$-$C_4$, en particulier le groupe méthyle ou éthyle, et le substituant -X-$R_3$ est en position ortho ou para.

4. Produit selon la revendication 1 ou 2, caractérisé par le fait qu'il contient en tant que composant (A) un composé de formule (1) dans lequel $R_4$ et $R_5$ sont en position 2 et en position 6 et représentent des atomes d'hydrogène ou des groupes alkyle en $C_1$-$C_4$, et -X-$R_3$ représente le groupement 4-trifluorométhylphénoxy.

5. Produit selon l'une des revendications 1 à 3, caractérisé par le fait qu'il contient en tant que composant (A) un composé de formule (1) dans lequel X représente un atome d'oxygène et $R_3$ représente un radical halogéno-alkyle en $C_1$-$C_4$.

6. Produit selon la revendication 1 ou 3, caractérisé par le fait qu'il contient en tant que composant (A) un composé de formule (1) dans lequel X représente un atome d'oxygène et $R_3$ représente un radical phényle substitué par 1 à 3, de préférence 1 ou 2 substituants choisis parmi des atomes d'halogène ou des groupes halogéno-alkyle en $C_1$-$C_4$ ou alkyle en $C_1$-$C_4$.

7. Produit selon la revendication 1, caractérisé par le fait qu'il contient en tant que composant (B) un composé de type pyréthrine de formule (2), dans lequel (A) représente un reste de formule

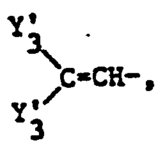

dans laquelle $Y'_3$ est Br, Cl ou $CH_3$; X représente un atome d'oxygène et Y représente

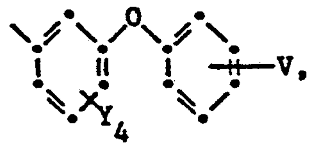

et $Y_2$ représente $CH_3$, et $Y_1$ représente un atome d'hydrogène, CN, $CH_3$, $-CH{=}CH_2$, $-C{\equiv}CH$ ou $-C{\equiv}C\text{-}CH_3$.

8. Produit selon l'une des revendications 1 à 7, caractérisé par le fait qu'il contient, en plus des composants (A) et (B), des véhicules usuels et/ou des produits auxiliaires de formulation usuels, par exemple des solvants organiquas, de l'eau, des acides, des bases, des agents mouillants, dispersants et/ou émulsifiants.

9. Produit selon la revendication 8, caractérisé par le fait qu'il contient, en plus des composants (A) et (B), une ou plusieurs amine(s) aliphatique(s) ou cycloaliphatique(s) et/ou un ou plusieurs amide(s) aliphatique(s) ou cycloaliphatique(s) et leur(s) dérivé(s), et éventuellement un ou plusieurs solvant(s) organique(s), de l'eau, un ou plusieurs agent(s) tensio-actif(s) et/ou émulsifiant(s) ou dispersant(s), et/ou un ou plusieurs acide(s) carboxylique(s) aliphatique(s).

10. Procédé pour la protection ou l'apprêtage de matériau à base de kératine, en particulier de la laine, de peaux, de plumes et de fourrures, avant ou contre l'attaque de parasites de la kératine, par exemple de larves de mites et de coléoptères, caractérisé par le fait que l'on traite le matériau à protéger avec une combinaison de substances actives constituée d'un ou de plusieurs 5-phénylcarbamylbarbiturate(s) de formule (1) ou de leurs formes tautomères et de leurs sels, et d'un ou de plusieurs composé(s) synthétique(s) de type pyréthrine de formule (2) ou (2a) selon la revendication 1.

11. Procédé selon la revendication 10, caractérisé par le fait que l'on utilise des composés de formule (1) dans lesquels X représente un atome d'oxygène; $R_1$ et $R_2$ représentent des groupes alkyle en $C_1$-$C_4$, en particulier le groupe méthyle ou éthyle, et le substituant $-X-R_3$ se trouve en position ortho ou para; et des composés synthétiques de type pyréthrine de formule (2) ou (2a), en particulier de formule (2), avant tout ceux dans lesquels A représente un reste de formule

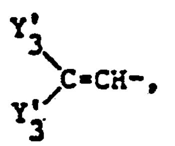

dans laquelle $Y'_3$ est Br, Cl ou $CH_3$; X représente un atome d'oxygène; et Y représente

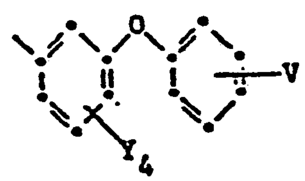

et $Y_2$ représente $CH_3$; et $Y_1$ représente un atome d'hydrogène, CN, $CH_3$, $-CH{=}CH_2$, $-C{\equiv}CH$ ou $-C{\equiv}C\text{-}CH_3$.

12. Procédé selon la revendication 10, caractérisé par le fait que l'on utilise un produit qui contient, en plus de la combinaison de substances actives, des véhicules usuels et/ou des produits auxiliaires de formulation usuels.

13. Procédé selon l'une des revendications 10 à 12, caractérisé par le fait que l'on traite le matériau à protéger, en particulier des textiles contenant des fibres de laine, avec un bain aqueux qui contient une combinaison de substances actives définie dans la revendication 12, le bain aqueux pouvant éventuellement contenir encore des produits auxiliaires pour textiles et/ou des colorants, ou on traite le matériau à protéger avec un liquide organique de nettoyage qui contient une combinaison de substances actives définie dans la revendication 12, ou on pulvérise sur le matériau à protéger un solvant organique qui contient une combinaison de substances actives définie dans la revendication 12.

14. Procédé selon l'une des rerevendications 10 à 13, caractérisé par le fait que l'on applique sur les matériaux à protéger la combinaison de substances actives en une quantité allant de 10 à 2 000 ppm, de préférence 100 à 1 000 ppm, par rapport au matériau à protéger.

15. Le matériau à base de kératine, de préférence textiles contenant de la laine, peaux et fourrures, apprêté selon l'une des revendications 10 à 14, contenant 10 à 2 000 ppm, de préférence 100 à 1 000 ppm, d'une

combinaison de substances activés définie dans la revendication 12.

16. Utilisation des produits définis dans la revendication 1 ou 2, pour la protection de matériau à base de kératine, en particulier de textiles de laine, contre l'attaque de parasites de la kératine.